# EUROPEAN PATENT APPLICATION

(11) **EP 2 823 759 A1**
(43) Date of publication of application: **14.01.2015**
(21) Application number: 13758178.1
(22) Date of filing: 06.03.2013
(51) Int. Cl.: A61B 5/0245

(54) **PULSE MONITOR AND PROGRAM**

(30) Priority: 07.03.2012 JP 2012050139; 27.03.2012 JP 2012070592
(71) Applicant: Seiko Epson Corporation, Shinjuku-ku Tokyo 163-0811 (JP)
(72) Inventor: TAKAHASHI, Yusuke, Suwa-shi Nagano 392-8502 (JP); KURODA, Masao, Suwa-shi Nagano 392-8502 (JP)
(74) Representative: Hoffmann Eitle
(86) International application number: PCT/JP2013/001390
(87) International publication number: WO 2013/132844

(57) **Abstract**

A pulse monitor, a program, and the like which perform determination of an appropriate pressing force and store and display holding state specification information when it is determined to be an appropriate pressing force, thereby facilitating device mounting in an appropriate state are provided. A pulse monitor includes a pulse wave detection unit 10 which has a pulse wave sensor 11 configured to output a pulse wave sensor signal, a processing unit 100 which calculates pulse information based on the signal from the pulse wave detection unit 10, a display unit 70 which displays a processing result in the processing unit 100, a storage unit 90 which stores the processing result in the processing unit 100, and a holding mechanism which holds the pulse monitor on a subject. The processing unit 100 determines whether or not a pressing force on the subject in the pulse wave detection unit 10 is an appropriate pressing force, the storage unit 90 stores holding state specification information for specifying the holding state of the holding mechanism when it is determined to be an appropriate pressing force, and the processing unit 100 performs control for displaying the holding state specification information on the display unit 70.

## Description

### Technical Field

The present invention relates to a pulse monitor, a program, and the like.

### Background Art

In the related art, an electronic apparatus, such as a pulse monitor, has come into wide use. The pulse monitor is a device which detects a pulse resulting from heartbeat of a human body, and is a device which detects a signal resulting from heartbeat based on a signal from a pulse wave sensor mounted on an arm, a palm, a finger, or the like.

As the pulse wave sensor, for example, a photoelectric sensor is used. In this case, a method which detects reflected light or transmitted light of light irradiated onto a living body by the photoelectric sensor, or the like is considered. Since the amount of absorption and the amount of reflection of irradiated light in the living body differ depending on the blood flow rate in a blood vessel, sensor information (pulse wave sensor signal) detected by the photoelectric sensor becomes a signal corresponding to the blood flow rate or the like, and the signal is analyzed, thereby acquiring information relating to a pulse.

However, it is known that the amplitude of a pulse signal is different depending on a pressing force (here, an external pressure which is pressure from the outside of a living body to the living body). Since the pulse signal is lowered at an extremely large pressing force or an extremely small pressing force, in order to perform processing (for example, calculation of pulse information, or the like) based on the pulse signal with higher precision, it is necessary to set an appropriate pressing force.

### Citation List

### Patent Literature

PTL 1: JP-A-2008-54890

### Summary of Invention

### Technical Problem

For example, PTL 1 presents a structure which can measure a contact pressure of a biological information detection unit configured to detect biological information, such as a pulse monitor. A method which determines whether or not a contact pressure is an appropriate pressing force and displays the pressure as graphical representation to give notification to the user is also presented.

However, a vital sign, such as a pulse, has an extremely large individual difference, and for this reason, the value of the appropriate pressing force is different for each user. Meanwhile, in the method of PTL 1, since processing based on simple physical information (for example, a physical quantity in units of kPa or the like) regarding pressure is merely performed and the individual difference or the like is not taken into consideration, it is not possible to reliably perform determination about whether or not the pressing force is appropriate. For example, even if the pressure falls within an appropriate pressing force range set in advance, this is meaningless when an actual pulse signal is extremely small.

Similarly, in giving notification to the user, since the relationship with pressure set in advance (without taking the individual difference into consideration) is merely provided as graphical representation, it is not possible to determine how about the SN ratio of the actual pulse signal.

In PTL 1, the appropriate pressing force is treated only as physical information (contact pressure), and the mounting state of the apparatus for realizing the contact pressure is not taken into consideration. For example, in a pulse monitor which is mounted on a living body by a band, information regarding how much the band is fastened to obtain a desired contact pressure is not held. Therefore, the user is forced to adjust the mounting state each time the apparatus is mounted, and this is undesirable in terms of convenience or the like.

According to several aspects of the invention, it is possible to provide a pulse monitor, a program, and the like which perform determination of an appropriate pressing force and store and display holding state specification information when it is determined to be the appropriate pressing force, thereby facilitating apparatus mounting in an appropriate state.

### Solution to Problem

A pulse monitor according to an aspect of the invention includes a pulse wave detection unit which has a pulse wave sensor configured to output a pulse wave sensor signal, a processing unit which calculates pulse information based on the signal from the pulse wave detection unit, a display unit which displays a processing result in the processing unit, a storage unit which stores the processing result in the processing unit, and a holding mechanism which holds the pulse monitor on a subject, in which the processing unit determines whether or not a pressing force on the subject in the pulse wave detection unit is an appropriate pressing force, the storage unit stores holding state specification information for specifying the holding state of the holding mechanism when it is determined that the pressing force on the subject in the pulse wave detection unit is the appropriate pressing force, and the processing unit performs control for displaying the holding state specification information on the display unit.

In the aspect of the invention, determination about whether or not the pressing force on the subject is the appropriate pressing force is performed, and information for specifying the holding state of the holding mechanism when the pressing forces is the appropriate pressing force is stored and displayed as the holding state specification information. Therefore, since the appropriate pressing force can be stored or displayed as the holding state, instead of physical information, such as a pressure value, it is possible to present information of the appropriate pressing force to the user in a simple form, and to facilitate reproduction of the holding state for realizing the appropriate pressing force, or the like.

In the aspect of the invention, the processing unit may perform determination about whether or not the pressing force is the appropriate pressing force based on the pulse wave sensor signal.

With this, it becomes possible to perform the appropriate pressing force determination or the like taking into consideration an individual difference of each user.

In the aspect of the invention, the processing unit may perform determination about whether or not the pressing force is the appropriate pressing force based on at least one of an AC component signal corresponding to an AC component of the pulse wave sensor signal and a DC component signal corresponding to a DC component of the pulse wave sensor signal.

With this, it becomes possible to perform the appropriate pressing force determination or the like using at least one of the AC component signal and the DC component signal.

In the aspect of the invention, the processing unit may perform determination about whether or not the pressing force is the appropriate pressing force based on the change characteristic of the AC component signal when the pressing force is changed.

With this, it becomes possible to perform processing without depending on the value, and to perform the appropriate pressing force determination or the like taking into consideration an individual difference.

In the aspect of the invention, the processing unit may perform determination about whether or not the pressing force is the appropriate pressing force based on the change characteristic in amplitude of the AC component signal when the pressing force is changed.

With this, it becomes possible to perform the appropriate pressing force determination based on the change characteristic of amplitude of the AC component signal.

In the aspect of the invention, the processing unit may perform determination about whether or not the pressing force is the appropriate pressing force based on the change characteristic of the DC component signal when the pressing force is changed.

With this, it becomes possible to perform processing without depending on the value, and to perform the appropriate pressing force determination or the like taking into consideration an individual difference.

In the aspect of the invention, the processing unit may detect an inflection point of the DC component signal based on the change characteristic of the DC component signal when the pressing force is changed and may perform determination about whether or not the pressing force is the appropriate pressing force based on the detected inflection point.

With this, it becomes possible to perform the appropriate pressing force determination based on the inflection point of the DC component signal.

In the aspect of the invention, the processing unit may perform control for displaying, on the display unit, an instruction screen for giving an instruction to set an environment for determination about whether or not the pressing force is the appropriate pressing force.

With this, since it is possible to give the instruction to set the environment for determination, it becomes possible to achieve improvement of precision of the appropriate pressing force determination.

In the aspect of the invention, the processing unit may perform control for displaying, on the display unit, a screen for giving an instruction to stabilize body motion of the subject as the instruction screen.

With this, it becomes possible to give the instruction to stabilize body motion as the instruction to set the environment for determination.

In the aspect of the invention, the processing unit may perform control for displaying, on the display unit, a screen for giving an instruction to the subject to take a given posture for determination as the instruction screen.

With this, it becomes possible to give the instruction to take the given posture for determination as the instruction to set the environment for determination.

In the aspect of the invention, the processing unit may perform control for displaying, on the display unit, a screen for giving an instruction to the subject to stay on standby for a given time as the instruction screen.

With this, it becomes possible to give the instruction to stay on standby for the given time as the instruction to set the environment for determination.

In the aspect of the invention, the processing unit may perform determination about whether or not the condition of the environment for determination displayed on the instruction screen is satisfied, and may perform determination about whether or not the pressing force is the appropriate pressing force when it is determined that the condition is satisfied.

With this, after the instruction to set the environment for determination is given, it becomes possible to perform determination about whether or not the instruction is satisfied, or the like.

In the aspect of the invention, the processing unit may perform determination about whether or not the condition of the environment for determination displayed on the instruction screen is satisfied based on at least one of a body motion detection signal from a body motion sensor and time measurement information from a time measurement unit, and may perform determination about whether or not the pressing force is the appropriate pressing force when it is determined that the condition is satisfied.

With this, it becomes possible to perform determination about whether or not the condition of the environment for determination is satisfied based on the body motion detection signal and the time measurement information.

In the aspect of the invention, the processing unit may have a holding state specification information acquisition mode and a pulse information calculation mode, in which the pulse information is calculated, as a processing mode, when the holding state specification information acquisition mode is set, the processing unit may acquire the holding state specification information based on the determination result about whether or not the pressing force is the appropriate pressing force and may store the acquired holding state specification information in the storage unit, and when the pulse information calculation mode is set after the holding state specification information is acquired, the processing unit may perform control for reading the holding state specification information stored in the storage unit and displaying the read holding state specification information on the display unit.

With this, in the pulse information calculation mode, it becomes possible to display the holding state specification information acquired in the holding state specification information acquisition mode, and to perform pulse information calculation or the like with high precision.

In the aspect of the invention, the holding mechanism may take first to N-th (where N is an integer equal to or greater than 2) states, in which the pressing force on the subject in the pulse wave detection unit is different, as the holding state, and the processing unit may acquire information corresponding to at least one of the first to N-th states as the holding state specification information based on a determination result about whether or not the pressing force is the appropriate pressing force in each of the first to N-th states.

With this, in the holding mechanism which takes a plurality of holding states, it becomes possible to acquire information corresponding to at least one of the plurality of holding states as the holding state specification information.

In the aspect of the invention, after determination about whether or not the pressing force is the appropriate pressing force in an i-th (where i is an integer which satisfies 1 ≤ i ≤ N) states, the processing unit may perform control for displaying, on the display unit, an instruction screen for giving an instruction to change the holding state in the holding mechanism to a j-th (where j is an integer which satisfies 1 ≤ j ≤ N and j ≠ i) state in which the pressing force is less than the i-th state.

With this, since it is possible to perform the appropriate pressing force determination in a depressurization process, and improve precision of the appropriate pressing force determination, or the like.

A pulse monitor according to another aspect of the invention includes a pulse wave detection unit which has a pulse wave sensor, a processing unit which determines whether or not a pressing force on a subject in the pulse wave detection unit is an appropriate pressing force and calculates pulse information of the subject based on a signal from the pulse wave detection unit, a display unit which displays a processing result in the processing unit, and a storage unit which stores the processing result in the processing unit, in which the processing unit performs control for displaying, on the display unit, an instruction screen for giving an instruction to set an environment for determination about whether or not the pressing force is the appropriate pressing force.

In the aspect of the invention, when performing the appropriate pressing force determination, the instruction screen for giving the instruction to set the environment for determination is displayed on the display unit. Therefore, since it can be expected that an environment suitable for the appropriate pressing force determination is set, it is possible to improve determination precision or the like.

In the aspect of the invention, the processing unit may perform determination about whether or not the condition of the environment for determination displayed on the instruction screen is satisfied and may perform determination about whether or not the pressing force is the appropriate pressing force based on the signal from the pulse wave detection unit when it is determined that the condition is satisfied.

With this, since it is possible to perform processing based on the signal from the pulse wave detection unit when the condition of the environment for determination is satisfied, it becomes possible to achieve improvement of determination precision, or the like.

According to still another aspect of the invention, there is provided a program which causes a computer to function as the above-described respective units.

### Brief Description of Drawings

[Fig. 1] Fig. 1 is a basic configuration example of a pulse monitor of a first embodiment.
[Fig. 2] Fig. 2 shows a configuration example of a body motion noise reduction unit using an adaptive filter.
[Fig. 3] Figs. 3(A) to 3(C) show examples of the waveforms and frequency spectrums of a pulse wave detection signal, a body motion detection signal, and a signal after body motion noise reduction processing based on these signals.
[Fig. 4] Figs. 4 (A) and 4 (B) show an example of a pulse monitor.
[Fig. 5] Fig. 5 shows a detailed configuration example of the pulse monitor of the first embodiment.
[Fig. 6] Fig. 6 is a relationship diagram between a pressing force and a pulse wave detection signal (AC component signal) .
[Fig. 7] Fig. 7 shows a configuration example of a band which is a holding mechanism.
[Fig. 8] Figs. 8 (A) and 8 (B) are relationship diagrams between a pressing force and an AC component signal.
[Fig. 9] Fig. 9(A) shows an example of a signal value of an AC component signal at a given pressing force, and Fig. 9(B) shows an example of an amplitude value of an AC component signal at a given pressing force.
[Fig. 10] Fig. 10 is a relationship diagram between a pressing force and a DC component signal.
[Fig. 11] Fig. 11 shows an example of a signal value of a DC component signal at a given pressing force.
[Fig. 12] Fig. 12 is a flowchart illustrating appropriate pressing force determination processing using both of an AC component signal and a DC component signal.
[Fig. 13] Fig. 13 is a flowchart illustrating processing of the embodiment including display control and the like.
[Fig. 14] Figs. 14(A) to 14(C) show a screen example which is displayed on a display unit.
[Fig. 15] Figs. 15 (A) and 15 (B) show an example of an acceleration detection value for use in body motion stability determination, and Fig. 15 (C) shows a setting example of axes of an acceleration sensor.
[Fig. 16] Fig. 16 is a flowchart illustrating body motion stability determination processing.
[Fig. 17] Figs. 17 (A) and 17 (B) show an example of an acceleration detection value for use in posture determination.
[Fig. 18] Fig. 18 is a flowchart illustrating posture determination processing.
[Fig. 19] Figs. 19(A) to 19(C) are diagrams illustrating a difference between change characteristics of an AC component signal and a DC component signal in a pressurization process and a depressurization process.
[Fig. 20] Figs. 20(A) to 20(C) are explanatory views showing when a pulse component is attenuated in body motion noise reduction processing of a second embodiment.
[Fig. 21] Fig. 21 shows a detailed configuration example of an electronic apparatus including a pulse detector of the second embodiment.
[Fig. 22] Fig. 22 is a flowchart illustrating the entire flow of processing of the second embodiment.
[Fig. 23] Figs. 23(A) to 23(C) are explanatory views showing when an acceleration detection signal is used in body motion noise reduction processing.
[Fig. 24] Fig. 24 is a flowchart illustrating the flow of fundamental frequency calculation processing.
[Fig. 25] Figs. 25(A) to 25(C) are diagrams showing an example of a detection result of a fundamental frequency.
[Fig. 26] Fig. 26 is a flowchart illustrating the flow of determination processing which performs time frequency analysis processing.
[Fig. 27] Fig. 27 is a flowchart illustrating the flow when a pulse wave detection signal is also used in the determination processing.

### Description of Embodiments

### (First Embodiment)

Hereinafter, an embodiment will be described. The embodiment described below is not intended to unduly limit the content of the invention described in the appended claims. It is not always the case that all configurations described in the embodiment are the essential constituent elements of the invention.

### 1-1. Configuration example of pulse monitor

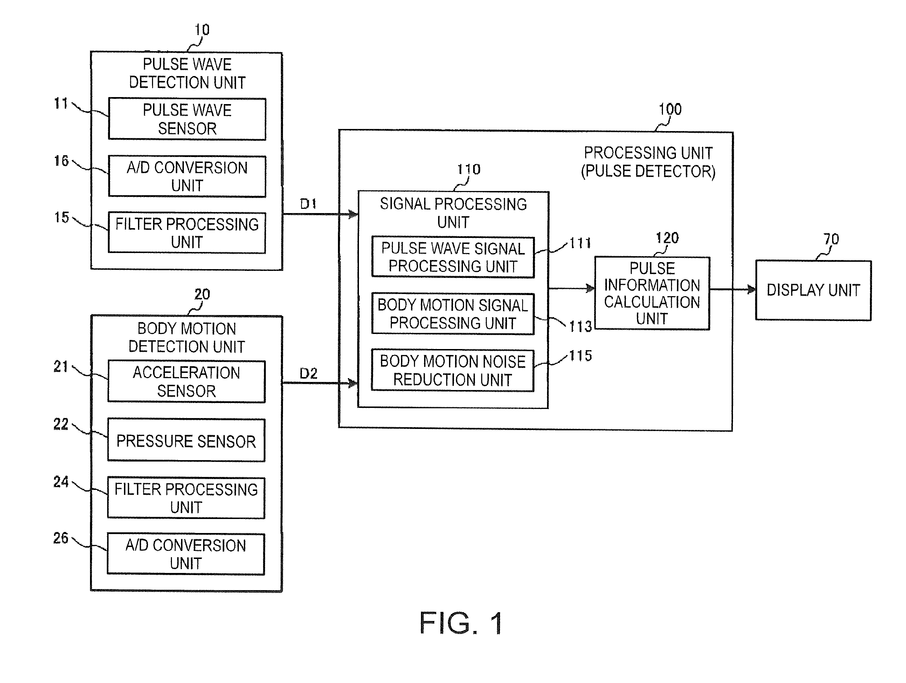

First, a basic configuration example of a pulse monitor (in a broad sense, an electronic apparatus) of the embodiment will be described referring to Fig. 1. Fig. 1 shows an example of the pulse monitor, and the configuration included in the pulse monitor of the embodiment may be simplified or omitted, or a configuration which is not essential in the pulse monitor of the embodiment may be included.

As shown in Fig. 1, the pulse monitor of the embodiment includes a pulse wave detection unit 10, a body motion detection unit 20, a processing unit 100, and a display unit 70. However, the pulse monitor is not limited to the configuration of Fig. 1, and various modifications may be made, in which some constituent elements may be omitted or changed, other constituent elements may be added, or the like.

The pulse wave detection unit 10 outputs a signal based on sensor information (pulse wave sensor signal) of a pulse wave sensor 11. The pulse wave detection unit 10 can include, for example, a pulse wave sensor 11, a filter processing unit 15, and an A/D conversion unit 16. However, the pulse wave detection unit 10 is not limited to the configuration of Fig. 1, and various modifications may be made, in which some constituent elements may be omitted, other constituent elements (for example, an amplification unit which amplifies a signal, and the like) may be added, or the like.

The pulse wave sensor 11 is a sensor which detects a pulse wave signal, and for example, a photoelectric sensor or the like is considered. When a photoelectric sensor is used as the pulse wave sensor 11, a sensor which is configured to cut a signal component of external light, such as sunlight, may be used. This can be realized by, for example, providing a plurality of photodiodes and performing feedback processing or the like using the signals of the photodiodes to obtain difference information.

The pulse wave sensor 11 is not limited to a photoelectric sensor, and may be a sensor which uses an ultrasonic wave. In this case, the pulse wave sensor 11 has two piezoelectric elements, excites one piezoelectric element to transmit an ultrasonic wave into a living body, and receives a reflected wave of the ultrasonic wave by the blood flow of the living body by the other piezoelectric element. Since there is frequency change between the transmitted ultrasonic wave and the received ultrasonic wave due to the Doppler effect of the blood flow, in this case, it is possible to acquire a signal corresponding to the blood flow rate, and to estimate pulse information. As the pulse wave sensor 11, other sensors may be used.

The filter processing unit 15 performs high pass filter processing for the sensor information from the pulse wave sensor 11. Note that the cutoff frequency of the high pass filter may be obtained from a typical pulse rate. For example, there are very few cases where a usual pulse rate of a person falls below 30 per minute. That is, since there are very few cases where the frequency of a signal resulting from heartbeat is equal to or lower than 0.5 Hz, even if information of a frequency band in this range is cut, there ought to be little adverse influence on a signal desired to be acquired. Accordingly, the cutoff frequency may be set to about 0.5 Hz. In some cases, a different cutoff frequency, such as 1 Hz, may be set. Furthermore, since a typical upper limit value to the pulse rate of the person can be assumed, the filter processing unit 15 may perform band pass filter processing, instead of high pass filter processing. Although a cutoff frequency on a high frequency side may be freely set to some extent, for example, a value of 4 Hz or the like may be used.

The A/D conversion unit 16 performs A/D conversion processing and outputs a digital signal. The processing in the above-described filter processing unit 15 may be analog filter processing which is performed before the A/D conversion processing or digital filter processing which is performed after the A/D conversion processing.

The body motion detection unit 20 outputs a signal (body motion detection signal) according to body motion based on the sensor information of various sensors. The body motion detection unit 20 can include, for example, an acceleration sensor 21, a pressure sensor 22, and an A/D conversion unit 26. However, the body motion detection unit 20 may include other sensors (for example, a gyro sensor), an amplification unit which amplifies a signal, or the like. It is not necessary to provide a plurality of types of sensors, and a configuration in which one type of sensor is included may be made.

The processing unit 100 includes a signal processing unit 110 and a pulse information calculation unit 120. However, the processing unit 100 is not limited to the configuration of Fig. 1, and various modifications may be made, in which some constituent elements are omitted, other constituent elements are added, or the like. The signal processing unit 110 performs signal processing for an output signal from the pulse wave detection unit or an output signal from the body motion detection unit.

The signal processing unit 110 can include a pulse wave signal processing unit 111, a body motion signal processing unit 113, and a body motion noise reduction unit 115.

The pulse wave signal processing unit 111 performs some kind of signal processing for a signal from the pulse wave detection unit 10. As the output from the pulse wave detection unit 10 represented by D1 of Fig. 1, various signals based on the pulse wave sensor signal are considered. For example, since there are many cases where the calculation of the pulse information described below is performed based on the pulse wave sensor signal (hereinafter, referred to as a pulse wave detection signal, and in the following description, an equivalent signal is referred to as an AC component signal) after DC component cutting, it is assumed that the pulse wave sensor signal after the high pass filter processing is included in D1. However, a signal which is not subjected to filter processing may be output, or in some cases, a pulse wave sensor signal after low pass filter processing may be output. When a plurality of signals (for example, both a pulse wave sensor signal before the high pass filter processing and a pulse wave sensor signal after the processing) are included in D1, the processing in the pulse wave signal processing unit 111 may be performed for all signals included in D1 or may be performed for some signals. Various processing contents are considered, and for example, equalizer processing for the pulse wave detection signal may be performed, or other kinds of processing may be performed.

The body motion signal processing unit 113 performs various kinds of signal processing for the body motion detection signal from the body motion detection unit 20. Similarly to D1, as the output from the body motion detection unit 20 represented by D2, various signals are considered. In the example of Fig. 1, since the acceleration sensor 21 and the pressure sensor 22 are provided, the body motion detection signal of D2 includes an acceleration signal and a pressure signal. Since other sensors, such as a gyro sensor, may be used as a body motion detection sensor, output signals corresponding to the types of sensors are included in D2. The processing in the body motion signal processing unit 113 may be performed for all signals included in D2 or may be performed for some signals. For example, comparison processing of the signals included in D2 may be performed, thereby performing processing for determining a signal for use in noise reduction processing in the body motion noise reduction unit 115.

Note that, in the processing in the pulse wave signal processing unit 111, the body motion detection signal may be used along with the signal from the pulse wave detection unit. Similarly, in the processing in the body motion signal processing unit 113, the signal from the pulse wave detection unit 10 may be used along with the body motion detection signal. A signal after given processing is performed for the output signal from the pulse wave detection unit 10 in the pulse wave signal processing unit 111 may be used for use in the processing in the body motion signal processing unit 113, or otherwise may be made.

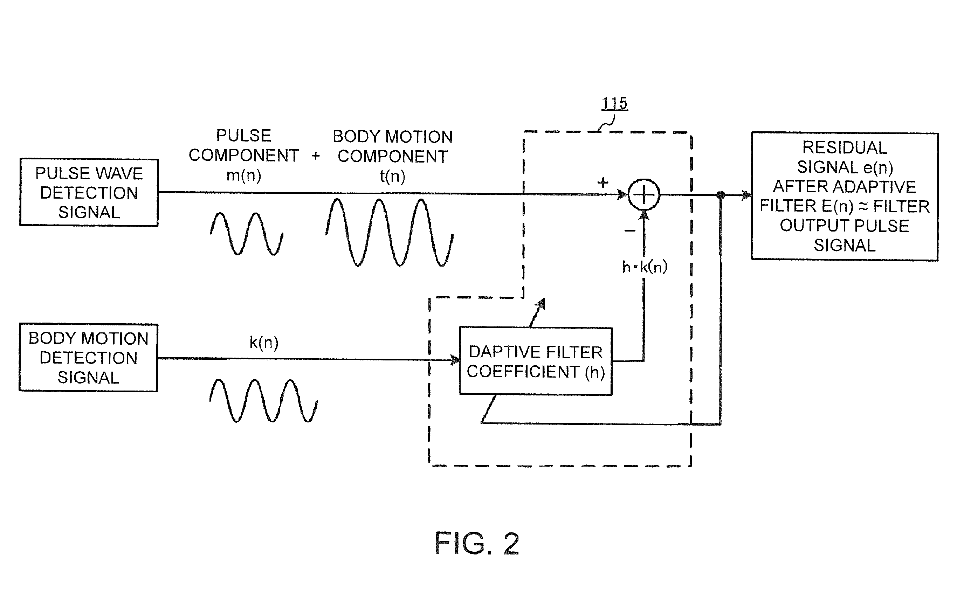

The body motion noise reduction unit 115 performs processing for reducing noise due to body motion (body motion noise) from the pulse wave detection signal using the body motion detection signal. A specific example of the noise reduction processing using an adaptive filter is shown in Fig. 2. The pulse wave sensor signal acquired from the pulse wave sensor 11 includes a component due to body motion in addition to a component due to heartbeat. The same applies to the pulse wave detection signal (the pulse wave sensor signal after DC component cutting) for use in pulse information calculation. Of these, a component due to heartbeat is useful for the calculation of the pulse information, and the component due to body motion is obstructive to the calculation. Accordingly, a signal (body motion detection signal) due to body motion is acquired using a body motion sensor, and a signal component (called an estimated body motion noise component) correlated to the body motion detection signal is removed from the pulse wave detection signal, thereby reducing body motion noise included in the pulse wave detection signal. However, even if body motion noise in the pulse wave detection signal and the body motion detection signal from the body motion sensor are signals due to the same body motion, the signal levels are not identical. Accordingly, filter processing in which a filter coefficient is adaptively determined is performed for the body motion detection signal, whereby the estimated body motion noise component is calculated, and the difference between the pulse wave detection signal and the estimated body motion noise component is taken.

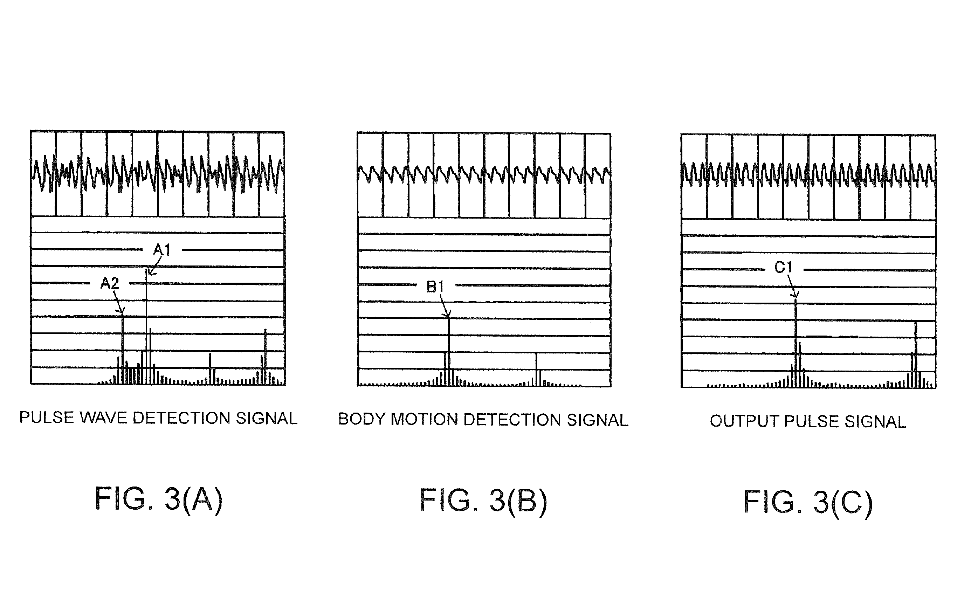

Figs. 3(A) to 3(C) illustrate the above-described processing by a frequency spectrum. In Fig. 3 (A) and the like, the time-varying waveform of a signal is shown on the upper side, and the frequency spectrum thereof is shown on the lower side. Fig. 3(A) shows a pulse wave detection signal before body motion noise reduction, and as indicated by A1 and A2, two frequencies having a large value appear in the spectrum. Of these, one frequency is due to heartbeat, and the other frequency is due to body motion. Although frequencies higher than A1 have a large value, since the frequencies are high-frequency components corresponding to an integer multiple of A1 and A2, in this case, these frequencies are not taken into consideration. Hereinafter, in Figs. 3(B) and 3(C), although high-frequency components are visible, similarly, in this case, these frequency components are not taken into consideration.

Meanwhile, Fig. 3(B) shows a body motion detection signal, and if one type of body motion is responsible for a body motion detection signal, as indicated by B1, a frequency having a large value appears. Here, the frequency of B1 corresponds to A2 of Fig. 3(A). In this case, the difference between the pulse wave detection signal and the estimated body motion noise component is taken by the method shown in Fig. 2, thereby obtaining a signal of Fig. 3 (C). As will be apparent from the drawing, the estimated body motion noise component having the peak B1 due to body motion is subtracted from the pulse wave detection signal having the two peaks A1 and A2 due to heartbeat and body motion, whereby a body motion component (corresponding to A2) in the pulse wave detection signal is excluded, and as a result, a peak C1 (whose frequency corresponds to A1) due to heartbeat remains.

When it is ensured that body motion noise included in the pulse wave detection signal corresponds to the body motion detection signal, no signal component which adversely affects the noise reduction processing is included in the body motion detection signal, or the like, since it is not necessary to perform frequency analysis in the body motion noise reduction unit 115, the frequency spectrum shown on the lower side of Figs. 3 (A) and 3 (B) may not taken into consideration. However, there is a case where the above-described condition is not satisfied depending on the type of a sensor which is used to acquire the body motion detection signal, or the like. In this case, for example, the body motion signal processing unit 113 may process the body motion detection signal such that the above-described condition is satisfied, or may exclude the body motion detection signal, which does not satisfy the above-described condition, from the output to the body motion noise reduction unit 115 or the like. Although various methods which perform determination about whether or not the above-described condition is satisfied are considered, for example, the frequency spectrum which is obtained by frequency analysis and shown on the lower side of Figs. 3(A) and 3(B) may be used.

The pulse information calculation unit 120 calculates pulse information based on an input signal. The pulse information may be, for example, the value of a pulse rate. For example, the pulse information calculation unit 120 may perform frequency analysis, such as FFT, for the pulse wave detection signal after the noise reduction processing in the body motion noise reduction unit 115 to obtain a spectrum, and may perform processing for setting a representative frequency in the obtained spectrum as the frequency of heartbeat. In this case, a value which is a 60th power of the obtained frequency becomes a pulse rate (heart rate) which is generally used.

The pulse information is not limited to the pulse rate, and may be, for example, information (the frequency of heartbeat, the cardiac cycle, or the like) representing the pulse rate. The pulse information may be information representing the state of a pulse, or for example, a value representing a blood flow rate (or variation in blood flow rate) may be used as the pulse information. However, since there is an individual difference in the relationship between the blood flow rate and the signal value of the pulse wave sensor signal between users, it is preferable to perform correction processing for coping with the individual difference when the blood flow rate or the like may be used as the pulse information.

The timing at which a given value (an upper peak, a lower peak, a value equal to or greater than a given threshold value, or the like) appears on the time-varying waveform of the input pulse wave detection signal may be detected, and the cardiac cycle may be obtained from the time corresponding to the interval of the timing to calculate the pulse information. Alternatively, the waveform of the pulse wave detection signal may be deformed to a rectangular wave, and the rising edge of the rectangular wave or the like may be used to calculate the pulse information. In this case, since frequency analysis may not be performed, it is excellent in terms of the amount of calculation or power consumption. However, in this method, since the signal value is used as it is without performing conversion to the frequency axis, it is necessary to adjust the waveform to some extent, thus, it is preferable that frequency analysis is performed when there is much noise or the like.

The display unit 70 (in a broad sense, an output unit) displays various display screens which are used to present the calculated pulse information or the like, and can be realized by, for example, a liquid crystal display, an organic EL display, or the like.

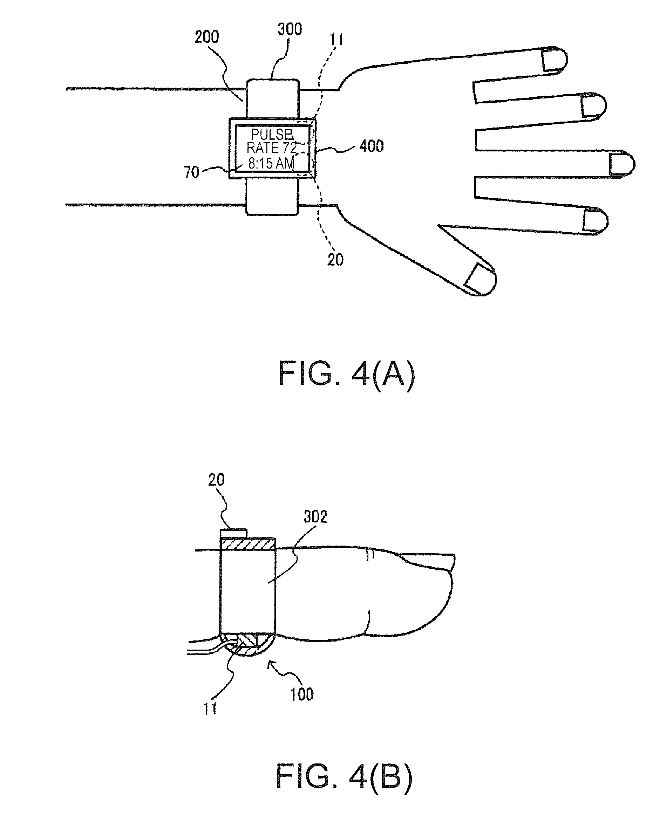

A specific example of the above-described pulse monitor is shown in Figs. 4(A) and 4(B). Fig. 4(A) shows an example of a wristwatch-type pulse monitor. A base portion 400 including the pulse wave sensor 11 and the display unit 70 is mounted on a left wrist 200 of the subject (user) by a holding mechanism 300 (for example, a band or the like). Fig. 4(B) shows an example of a finger-mounted type. The pulse wave sensor 11 is provided in the bottom portion of a ring-shaped guide 302 which is inserted into the fingertip of the subject. However, in the case of Fig. 4 (B), since there is no space margin for providing the display unit 70, it is assumed that the display unit 70 is provided (and a portion corresponding to the processing unit 100 as necessary), for example, on the other end side of a wire cable connected to the pulse wave sensor 11.

Although a method of the embodiment described below can be applied to any type of pulse monitor, it is more preferable that the method is applied to a wristwatch-type pulse monitor (the example of Fig. 4(A)). However, in the example of Fig. 4(A), the pulse wave sensor 11 is mounted in a region, such as the outside of the wrist (a region in contact with the rear cover surface of the wristwatch), in which a pulse wave sensor signal is difficult to acquire. For this reason, the amplitude of the pulse wave sensor signal output from the pulse wave sensor 11 generally tends to decrease. Accordingly, it is preferable to perform processing relating to precision of pulse information by any method.

### 1-2. Method of the embodiment

As described above, the pulse wave sensor, such as a photoelectric sensor, is used, whereby it is possible to acquire a pulse wave sensor signal corresponding to a blood circulation state (for example, a blood flow rate). However, as will be easily understood from the fact that, when the arm or the like is strongly compressed, the blood flow rate more decreases in a region on the distal side than the compressed portion, the blood flow rate changes depending on an external pressure on a living body (in a narrow sense, a blood vessel) . That is, if the external pressure is extremely strong, since the signal value of the pulse wave sensor signal decreases and the influence of noise relatively increases (an SN state is deteriorated), subsequent processing is hindered (for example, precision of pulse information based on the pulse wave sensor signal is degraded).

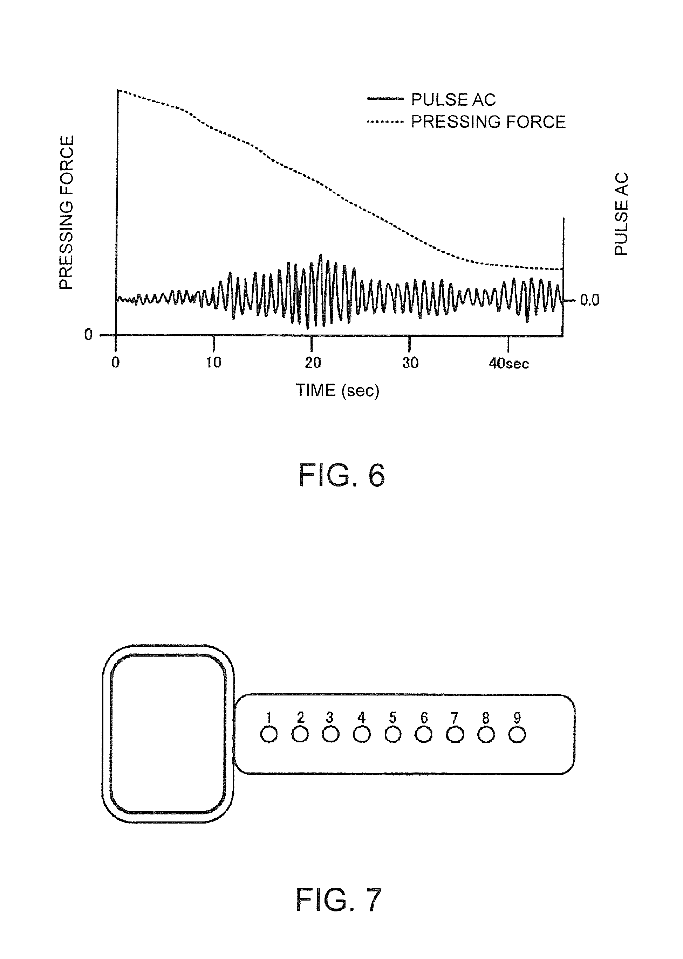

An extremely small external pressure is undesirable as well because the signal value of the pulse wave sensor signal decreases. As one of the factors of the decrease in the signal value, the influence of a component due to a vein is considered. Although the pulse wave sensor acquires both of a component due to an artery and a component due to a vein, a method which is in wide use is a method of performing calculation of pulse information based on the arterial component, or the like, and the venous component causes an adverse influence, such as a decrease in the signal value of the pulse wave sensor signal. That is, the extremely small external pressure is considered to be undesirable because the venous component affects and the signal value of the pulse wave sensor signal decreases. A change characteristic example of the AC component signal (the pulse wave detection signal) with respect to the external pressure is shown in Fig. 6. As will be apparent from Fig. 6, it is understood that the signal value decreases when the pressing force is extremely large or extremely small.

The blood flow decreases in both of the artery and the vein when the external pressure is applied thereto. From the characteristics of the living body, it is known that the blood flow sufficiently decreases in the vein with the external pressure less than the external pressure applied to the artery. That is, when pressure at a point where the blood flow sufficiently decreases (in a narrow sense, vanishes) in the vein (hereinafter, referred to as a venous vanishing point) is represented as V1 and pressure at a point where the blood flow sufficiently decreases in the artery (hereinafter, referred to as an arterial vanishing point) is represented as V2, the relationship of V1 < V2 is established. In this case, a situation in which the external pressure is greater than V2 is equivalent to the situation in which the external pressure is extremely strong. Even an arterial component originally desired to be acquired vanishes and a sufficient signal value cannot be obtained. On the other hand, when the external pressure is less than V1, this is equivalent to the situation in which the external pressure is extremely small. A signal value is influenced by a venous component and a sufficient signal value is not obtained either.

That is, an external pressure V satisfying V1 < V <V2 is applied to the subject, making it possible to sufficiently suppress the influence of the venous component and to prevent the blood flow of the arterial component from decreasing more than necessary. In the embodiment, V satisfying the above-described condition is set as the appropriate pressing force. The appropriate pressing force may indicate a range of a part of V, a specific pressure value, or the like, instead of the whole of V satisfying the above-described condition.

For example, a method which measures a contact pressure in a portion where biological information is detected using a pressure sensor or the like and presents whether or not a current pressure is an appropriate pressing force to the user as graphical representation through comparison processing of the contact pressure and a given reference value, or the like has been disclosed. However, a vital sign, such as a pulse, has an extremely large individual difference. The appropriate pressing force has a different value (range) for each user. Therefore, whereas it is not possible to cope with the individual difference unless the reference value for comparison with the measured contact pressure is determined for each user, in the method of the related art, processing based on simple physical information regarding pressure is merely described. It is unlikely that a signal due to the individual difference is superimposed on the physical information of the pressure. For example, a method of setting a reference value corresponding to the individual difference, or the like is not disclosed. This means that a method of coping with the individual difference is not disclosed in the method of the related art.

Therefore, in the method of the related art, even if the adjustment of the magnitude of the external pressure is instructed to the user through the graphical representation or the like, since the appropriate pressing force as a reference cannot cope with the individual difference, it is unknown whether or not a result obtained by adjusting the magnitude of the external pressure in response to the instruction is an appropriate state. This point is considered to be a first problem.

A second problem in the method of the related art is that the correspondence relationship between the contact pressure and the holding state (mounting state) of the apparatus is not described. That is, even if the pressure value of the appropriate pressing force is known, it is not known how the apparatus is mounted to obtain the pressure value. After the apparatus is mounted on the user in a certain state and the contact pressure is measured, while an instruction to raise or lower the pressure therefrom (for example, to fasten or unfasten the band) can be given, if information of the contact pressure is not acquired once, it is not possible to start the instruction to begin with. Therefore, it is necessary to adjust the mounting state every time when the apparatus is mounted again.

Therefore, the applicant proposes a method of determining an appropriate pressing force based on a pulse wave sensor signal. Since the characteristic of the pulse wave sensor signal appears for each user, it is possible to cope with an individual difference if determination based on the pulse wave sensor signal is performed. Furthermore, although the range of the appropriate pressing force could fluctuate according to change in physical condition or the like even for the same user, the method in the embodiment can cope with the change.

However, as described above, since the signal value of the pulse wave sensor signal is different for each user, the signal value at the appropriate pressing force is comparatively large for some users and is comparatively small for other users. Therefore, even if a signal value at a certain pressing force is separately used, it is difficult to perform determination about whether or not the pressing force is the appropriate pressing force. Therefore, in the embodiment, it is assumed that the pulse wave sensor signal is acquired while changing a pressing force and determination is performed about whether or not the pressing force is the appropriate pressing force using the change characteristic of the pulse wave sensor signal with respect to the pressing force.

In the embodiment, when performing determination about whether or not the pressing force is an appropriate pressing force, the holding state specification information when it is determined to be the appropriate pressing force is stored. This is, for example, information indicating which band hole should be used to mount the apparatus to enable the appropriate pressing force to be applied for the user. With this, since an appropriate mounting state can be defined by, for example, the degree of fastening of the band rather than the value of the pressing force, this method is intuitive and clear for the user. If the stored holding state specification information is displayed, the user can easily reproduce the holding state for realizing the appropriate pressing force. That is, adjustment or the like is not required when the apparatus is mounted again, and there is an advantage in terms of convenience or the like.

In the method of the related art, a method of automatically performing pressurization and depressurization using a pump or the like is also disclosed. This method is preferable in that manual mounting state adjustment by the user is not required. However, when it is assumed that the apparatus is used in a daily life and during exercising like a wrist-mounted apparatus, this method is unrealistic if size, power consumption, and the like are considered. Therefore, this method is not taken into consideration. That is, in the embodiment, although the pressing force is changed in the determination of the appropriate pressing force as described above, it is assumed that the pressing force is manually changed by the user. Therefore, in order to instruct the user to appropriately change the pressing force, it is important to perform interaction with the user using an interface, such as the display unit.

Hereinafter, after a system configuration example of the pulse monitor is described, a method of determining an appropriate pressing force based on a pulse wave sensor signal will be described. Finally, a display control method when interaction with the user is assumed will be described.

It is known that the blood circulation state strictly depends on not only the external pressure but also an internal pressure which is the pressure inside a blood vessel. However, in the embodiment, as described below, control for displaying an appropriate instruction screen is performed to suppress fluctuation in internal pressure as much as possible, and the external pressure is changed in this state to perform processing, such as the determination of the appropriate pressing force. Therefore, in the method of the embodiment, a case where the internal pressure largely changes is an exceptional situation, and this case is not particularly taken into consideration. For this reason, the term "pressing force" of course means the external pressure, and even when the term "pressure" or "contact pressure" is used without any special notice, the term indicates the external pressure.

### 1-3. System configuration example

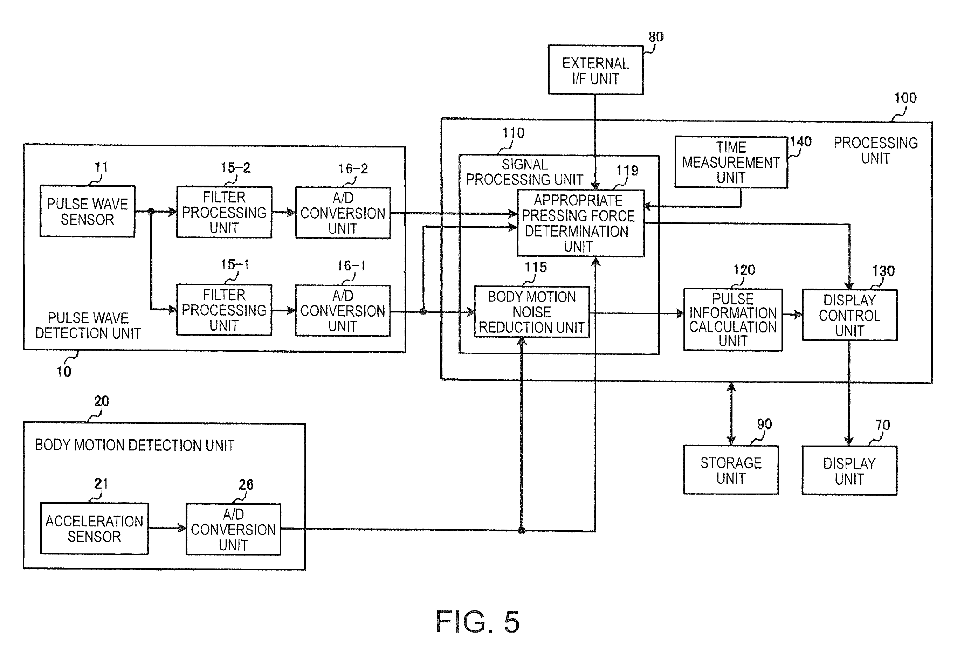

Fig. 5 shows a system configuration example of a pulse monitor of the embodiment. The pulse monitor includes a pulse wave detection unit 10, a body motion detection unit 20, a processing unit 100, a display unit 70, an external I/F unit 80, and a storage unit 90. However, the pulse monitor is not limited to the configuration of Fig. 5, and various modifications may be made, in which some constituent elements are omitted, other constituent elements are added, or the like. For example, in the embodiment, reduction in body motion noise is not essential, and the body motion noise reduction unit 115 and the like of the processing unit 100 may be omitted. As the overall configuration of the pulse monitor, as shown in Fig. 4(A), a configuration including the holding mechanism 300 and the base portion 400 is considered, and it is assumed that the respective units shown in Fig. 5 are included in the base portion 400 of Fig. 4(A).

The pulse wave detection unit 10 includes a pulse wave sensor 11, filter processing units 15-1 and 15-2, and A/D conversion units 16-1 and 16-2. However, the pulse wave detection unit 10 is not limited to the configuration of Fig. 5, and various modifications may be made, in which some constituent elements are omitted, other constituent elements are added, or the like.

As described referring to Fig. 1, as the pulse wave sensor 11, a photoelectric sensor or the like is used. In the embodiment, the filter processing unit 15-1 is realized by a high pass filter which performs high pass filter processing, and the filter processing unit 15-2 is realized by a low pass filter which performs low pass filter processing. That is, the output of the filter processing unit 15-1 is an AC component signal which is a high-frequency component of the pulse wave sensor signal, and the output of the filter processing unit 15-2 is a DC component signal which is a low-frequency component of the pulse wave sensor signal. In the embodiment, the pulse wave detection unit 10 includes the A/D conversion unit 16-1 and the A/D conversion unit 16-2 which respectively convert input analog signals into digital signals and output the digital signals.

As shown in Fig. 5, the pulse wave sensor 11 is connected to the filter processing unit 15-1 and the filter processing unit 15-2. The filter processing unit 15-1 is connected to the A/D conversion unit 16-1. The A/D conversion unit 16-1 is connected to the body motion noise reduction unit 115 and an appropriate pressing force determination unit 119 described below. The filter processing unit 15-2 is connected to the A/D conversion unit 16-2. The A/D conversion unit 16-2 is connected to the appropriate pressing force determination unit 119.

In the pulse wave detection unit 10, the filter processing unit 15-2 may be omitted. In this case, the output of the A/D conversion unit 16-2 becomes a signal which includes both a high-frequency component and a low-frequency component of a pulse wave sensor signal. Besides, in regard to the connection of the respective units in the pulse wave detection unit 10, various modifications may be made.

The body motion detection unit 20 includes an acceleration sensor 21 and an A/D conversion unit 26. The acceleration sensor 21 is connected to the A/D conversion unit 26, and the A/D conversion unit 26 is connected to the body motion noise reduction unit 115 and the appropriate pressing force determination unit 119. The body motion detection unit 20 may have a sensor which detects body motion, and the acceleration sensor 21 may be changed to a different sensor or may have a plurality of sensors.

The processing unit 100 includes a signal processing unit 110, a pulse information calculation unit 120, a display control unit 130, and a time measurement unit 140, and the signal processing unit 110 includes a body motion noise reduction unit 115 and an appropriate pressing force determination unit 119. However, the processing unit 100 and the signal processing unit 110 are not limited to the configuration shown in Fig. 5, and various modifications may be made, in which some constituent elements (for example, the body motion noise reduction unit 115) are omitted, other constituent elements are added, or the like.

The appropriate pressing force determination unit 119 determines whether or not a pressing force corresponding to the signal acquisition timing is an appropriate pressing force based on at least one of the AC component signal output from the A/D conversion unit 16-1 and the DC component signal output from the A/D conversion unit 16-2. At this time, a body motion detection signal output from the body motion detection unit 20, time measurement information output from the time measurement unit 140, or the like may be used. The appropriate pressing force determination unit 119 acquires holding state specification information for specifying the holding state of the holding mechanism 300 when it is determined that the pressing force is the appropriate pressing force based on, for example, information output from the external I/F unit 80 and outputs the acquired holding state specification information to the storage unit 90 and the display control unit 130. The details of the processing in the appropriate pressing force determination unit 119 will be described below.

The body motion noise reduction unit 115 performs body motion noise reduction processing for the AC component signal output from the A/D conversion unit 16-1 based on the body motion detection signal output from the body motion detection unit 20. The processing contents of the body motion noise reduction unit 115 are the same as the processing contents described above referring to Fig. 2 and the like, thus, detailed description of the processing contents will not be repeated. Processing in the pulse information calculation unit 120 is also as described above.

The display control unit 130 performs display control on the display unit 70. For example, in the determination in the appropriate pressing force determination unit 119, it is necessary to change the pressing force, and control for displaying an instruction screen for giving an instruction to the user is performed such that appropriate change in pressing force can be realized. Furthermore, control for displaying an instruction screen for giving an instruction to set an environment for determination about whether or not the pressing force is an appropriate pressing force may be performed. Besides, display control of the pulse information calculated by the pulse information calculation unit 120, or the like may be performed. The details will be described below.

The time measurement unit 140 performs time measurement processing. For example, a time measurement unit which has a timer configured to acquire time information, such as a time stamp, at a given interval and measures time from the difference of the acquired time information, or the like is considered.

The display unit 70 displays various kinds of information according to the control contents in the display control unit 130. The external I/F unit 80 functions as an interface with the outside, and in a narrow sense, the external I/F unit 80 may be an operating unit having various buttons and a GUI for the user to perform various kinds of operation of the pulse monitor. The storage unit 90 functions as a work area of the processing unit 100 and the like. A function of the storage unit 90 can be realized by a memory, such as a RAM or a HDD (hard disk drive). The storage unit 90 stores various kinds of information, and in particular, stores the holding state specification information acquired in the appropriate pressing force determination unit 119.

### 1-4. Determination of appropriate pressing force based on pulse wave sensor signal

### 1-4.1 Determination of appropriate pressing force

Next, a method of determining an appropriate pressing force based on a pulse wave sensor signal will be described. As described above, since a signal value of a pulse wave sensor signal is different depending on a user, a signal value at the appropriate pressing force is comparatively large for some users and is comparatively small for other users. Therefore, even if only a signal value at a given pressing force is acquired, it is difficult to perform determination about whether or not the pressing force is the appropriate pressing force based on the signal value. For example, even if it is attempted to perform determination about whether or not the pressing force is the appropriate pressing force by comparison processing of a signal value and a given threshold value, it is difficult to set a threshold value which can be generally used for a plurality of users.

Accordingly, in the embodiment, a pressing force on a living body is changed, and the appropriate pressing force is determined based on the change characteristic of a pulse wave sensor signal with respect to change in pressing force. This is because, while the magnitude of the signal value has an individual difference, the change characteristic of the pulse wave sensor signal has the same tendency in any user. For example, when discrete change in pressing force is considered, first to M-th different pressing forces are sequentially applied to a living body and pulse wave sensor signals at the respective pressing forces are acquired. The determination about which pressing force among the first to M-th pressing forces is the appropriate pressing force may be performed based on the acquired first to M-th pulse wave sensor signals.

Since the appropriate pressing force has a certain range (a range satisfying V1 < V < V2 described above) taking into consideration the characteristics of the living body, a pressing force which is determined as the appropriate pressing force according to the embodiment is not limited to one pressure value, the pressing force may have a plurality of values or may be represented by a given range.

In the following description, as shown in Fig. 4(A), a pulse monitor is a wristwatch-type apparatus having a band as the holding mechanism 300. As shown in Fig. 7, a plurality of holes are provided in the band. It is assumed that a pressing force can be changed according to which of the holes is used to hold the pulse monitor. However, the configuration of the holding mechanism 300 is not limited thereto.

Although a pressing force is changed by the user, in order to realize change in pressing force, display control for displaying an instruction screen on the display unit 70, or the like is performed. A specific interaction based on the display control or the like is described below. Here, determination processing based on an acquired pulse wave sensor signal will be described assuming that change in pressing force is appropriately performed. For improvement of determination precision, it is necessary to set an appropriate environment for determination. The environment setting is also performed by the display control of the instruction screen or the like. The details of the environment setting will be described below. Like change in pressing force, it is assumed that an appropriate environment for determination is set.

As described above, in the embodiment, processing is performed with the appropriate pressing force associated with the holding state of the holding mechanism 300 rather than a physical quantity in units of kPa or the like. Therefore, in the following description, for simplification of description, while a pressing force is changed to determine the appropriate pressing force, change in pressing force is equivalent to change in the holding state of the holding mechanism 300. The determination of the appropriate pressing force is equivalent to the determination of the holding state for realizing the appropriate pressing force.

### 1-4.2 Determination of appropriate pressing force based on AC component signal

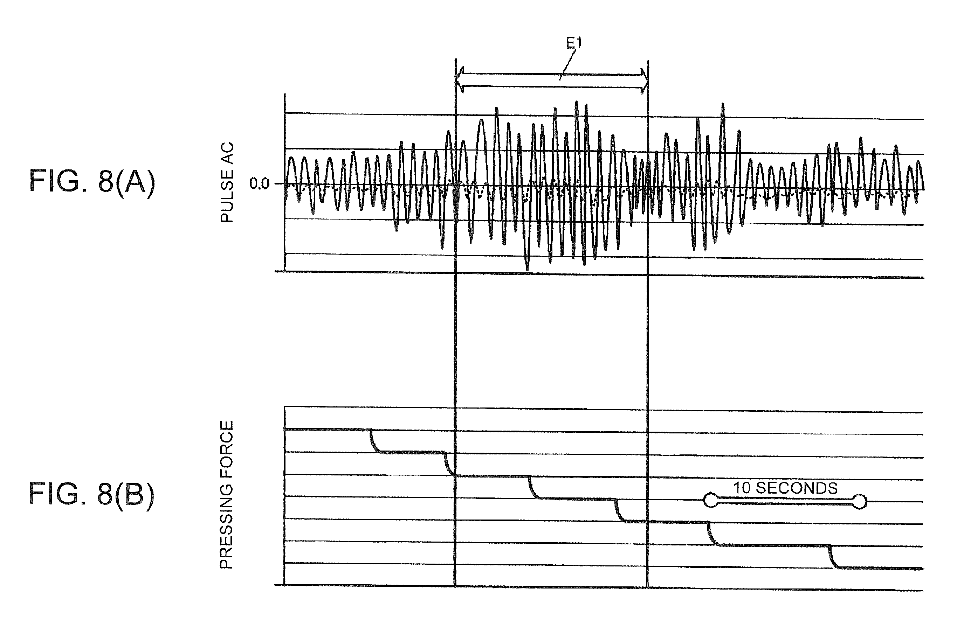

Determination based on an AC component signal corresponding to an AC component of a pulse wave sensor signal will be described. The change characteristic of the signal value of the AC component signal with respect to change in pressing force change is shown in Figs. 8(A) and 8(B). Fig. 8(A) is a diagram illustrating a general change tendency of the AC component signal with respect to the pressing force. The horizontal axis of Figs. 8(A) and 8(B) represents time. As will be apparent from temporal change in pressing force, Figs. 8 (A) and 8 (B) are graphs in a depressurization direction in which the pressing force is lowered along with the time.

As shown in Fig. 8(A), while the amplitude of the AC component signal has a small value when the pressing force is large, the amplitude value increases as the pressing force is lowered. When the pressing force is less than a given value, the amplitude value changes to a decreasing tendency. Considering the fact that the AC component signal is a signal due to heartbeat and is used for calculation of pulse information, a pressing force having a large amplitude value of the AC component signal may be set as the appropriate pressing force. For example, in Fig. 8(A), a range indicated by E1 becomes the appropriate pressing force.

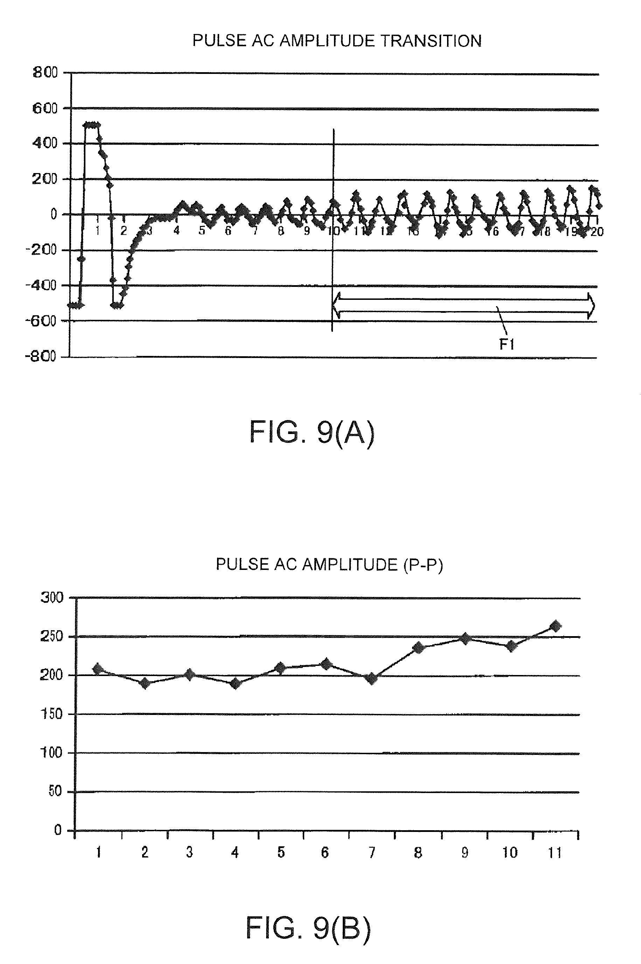

That is, when the AC component signal is used, amplitude values at the pressing forces may be calculated and a pressing force having a large calculated amplitude value may be set as the appropriate pressing force. A specific example is shown in Figs. 9(A) and 9(B). Fig. 9(A) shows temporal change in the AC component signal when a band hole position is set at a given position, and the horizontal axis thereof is in units of seconds. A first half portion (a period of 0 second to about 10 seconds) of Fig. 9(A) is the timing when the band hole position is set and a period in which the elapsed time from the timing is short. Since the signal value of the AC component signal is unstable during this period, the calculation of an amplitude value is not performed. That is, the amplitude value is calculated based on a signal value after a given time has elapsed after the band is mounted (for example, a signal value during a period of F1 of Fig. 9(A)).

The amplitude value may be calculated based on a peak. While either an upper peak or a lower peak may be used, here, it is assumed that the amplitude value is calculated from both of the upper peak and the lower peak. Specifically, a maximum value (the upper peak) and a minimum value (the lower peak) in one cycle of the AC component signal (corresponding to motion of one beat of the heart) are detected, and the difference value (peak to peak) between the maximum value and the minimum value is set as an amplitude value in the cycle. As indicated by F1 in Fig. 9(A), since it is assumed that the calculation period for the amplitude value is longer than one cycle of the AC component signal, a plurality of difference values are acquired in the calculation period. Fig. 9(B) shows an example of temporal change in difference value, and shows a state where difference values acquired once in each cycle of a pulse are arranged in an acquisition order (the horizontal axis represents the acquisition order and is not in units of seconds or the like). In the embodiment, the mean value of the plurality of difference values in the amplitude value calculation period (F1) may be set as an amplitude value at the set band hole position (and a pressing force corresponding to the band hole position).

The mean value may be a simple mean value or may be a trimmed mean value in which extremely large (or small) data is excluded in the mean value calculation. If the trimmed mean value is used, an exclusion range may be set from a standard deviation σ or the like, and for example, 3σ may be used.

With the above-described processing, it is possible to calculate an amplitude value of the AC component signal at a given pressing force (band hole position). In the determination of the appropriate pressing force, amplitude values at the pressing forces may be respectively calculated and a pressing force having a maximum amplitude value may be set as the appropriate pressing force.

### 1-4.3 Determination of appropriate pressing force based on DC component signal

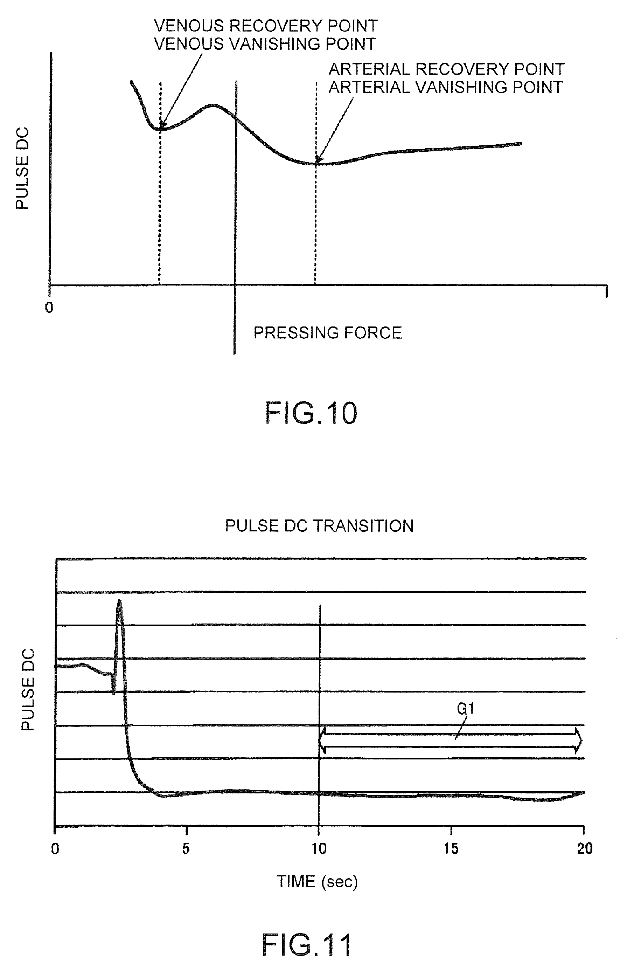

Determination based on a DC component signal corresponding to a DC component of a pulse wave sensor signal will be described. Fig. 10 shows the change characteristic of the signal value of a DC component signal with respect to change in pressing force. However, Fig. 10 is a diagram illustrating a general change tendency of a DC component signal with respect to a pressing force. Unlike Figs. 8 (A) and 8(B), the horizontal axis of Fig. 10 represents a pressing force.

As shown in Fig. 10, in a change characteristic curve of the DC component signal with respect to change in pressing force, both of a pressing force corresponding to a venous vanishing point and a pressing force corresponding to an arterial vanishing point are inflection points. That is, the inflection points of the DC component signal is detected, thereby detecting the pressing force corresponding to the venous vanishing point and the pressing force corresponding to the arterial vanishing point. As described above, since the appropriate pressing force may be pressure greater than the pressing force at the venous vanishing point and less than the pressing force at the arterial vanishing point, the appropriate pressing force may be determined within a range satisfying the condition. Within the pressure range satisfying the above-described condition, one pressure value may be set as the appropriate pressing force, and for example, the mean value of the pressing force at the venous vanishing point and the pressing force at the arterial vanishing point may be set as the appropriate pressing force.

Various methods of detecting the inflection point of the change characteristic curve of the DC component signal are considered. For example, as shown in Fig. 8 (B), when a pressing force changes over time, since a temporal change curve (that is, a graph in which the horizontal axis represents time and the vertical axis represents the signal value of the DC component signal) of the DC component signal corresponding to change in pressing force also represents the change characteristic of the DC component signal corresponding to change in pressing force, the inflection points of the curve may be detected. According to this method, since the signal values of the DC component signal acquired at the respective time can be directly used, it is advantageous in that preprocessing or the like is not required.

In the above-described temporal change curve, since the horizontal axis of the graph represents the time, instead of the pressing force, when change in the horizontal axis direction is considered, change does not correspond to linear change in pressing force. For example, when a pressing force during a period of the time T1 to the time T2 is considered, it is less likely that the pressing force changes at constant inclination. It is considered that the pressing force during the period is invariable or extremely changes to be a first pressing force during a period of T1 to T3 (T3 < T2) and a second pressing force during a period of T3 to T2. That is, it is expected that the change characteristic to the pressing force markedly appears when a pressing force change curve shown in Fig. 10 (a graph in which the horizontal axis represents the pressing force and the vertical axis represents the signal value of the DC component signal) is used rather than the temporal change curve. As in the description of the AC component signal, although it is considered that a signal value is not stable for a given time after the band is mounted, in the temporal change curve, since an unstable signal value directly appears, it is likely that there is an adverse influence on the processing.

Accordingly, in the embodiment, it is assumed that, when a given pressing force is set, one representative value is calculated based on the DC component signal at the pressing force. Since the processing is performed at a plurality of pressing forces to acquire a graph (the above-described pressing force change curve) which represents the corresponding relationship between the pressing force and the DC component signal, the inflection points in the acquired graph may be detected.

A method of calculating a representative value when the given pressing force is set will be described referring to Fig. 11. Fig. 11 represents temporal change in the DC component signal when a band hole position is set at a given position. As in Fig. 9(A), a first half portion of Fig. 11 is the timing when the band hole position is set and a period in which the elapsed time from the timing is short. Since the signal value of the DC component signal is unstable during this period, the calculation of a representative value is not performed, and a subsequent period indicated by G1 is used. While various methods of calculating a representative value are considered, for example, the mean value of the signal values of the DC component signal in a calculation section (G1 in Fig. 11) may be calculated. As in the processing on the AC component signal, the mean value may be a simple mean value or a trimmed mean value in which extremely large (or small) data is excluded in the mean value calculation. In this way, the representative values at the respective pressing forces are calculated, whereby it is possible to calculate the pressing force change curve of the DC component signal.

Detection of inflection points from a curve is a mathematically important field, and there are many examples of the related art regarding inflection point detection in a computer system or the like. In the embodiment, an arbitrary method among these examples may be used, thus, detailed description of a method of detecting inflection points will not be repeated.

### 1-4.4 Appropriate pressing force determination based on AC component signal and DC component signal

The appropriate pressing force determination of the embodiment may be processing based on one of an AC component signal and a DC component signal or may be processing using both signals.

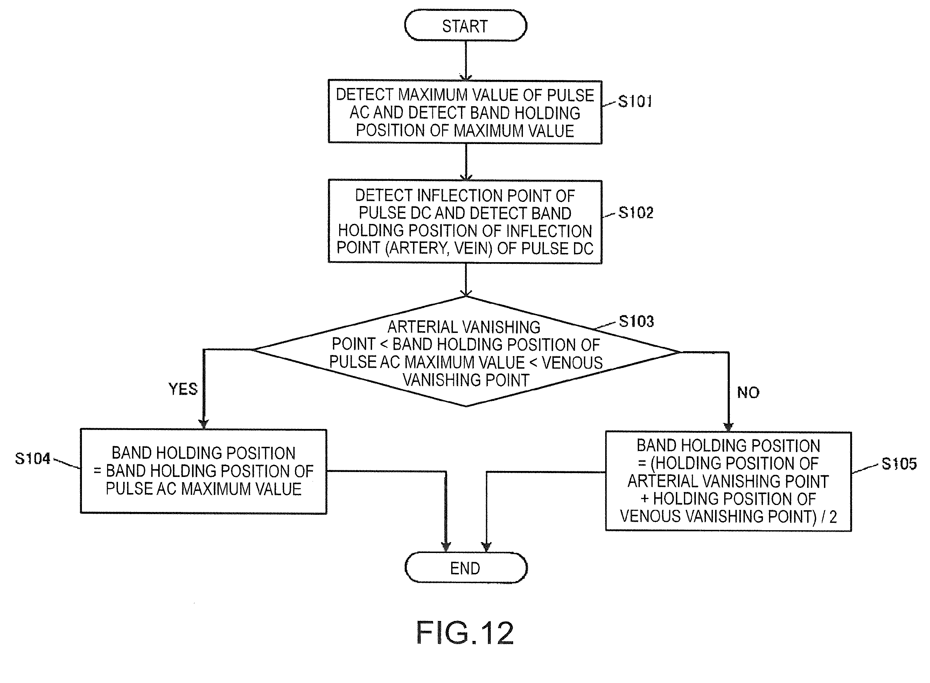

A specific example is shown in the flowchart of Fig. 12. If the processing starts, first, a pressing force VA having a maximum amplitude value is obtained based on an AC component signal (S101). Inflection point detection of a DC component signal is performed, thereby obtaining a pressing force VD1 at a venous vanishing point and a pressing force VD2 at an arterial vanishing point (S102). The specific processing of S101 and S102 are as described above.

Thereafter, determination is performed about whether or not the relationship of VD1 < VA < VD2 is established (S103), and when it is determined in S103 to be Yes, since VA falls within an appropriate range obtained from the DC component signal, reliability of VA is secured and VA is set to an appropriate pressing force (S104). When it is determined in S103 to be No, since reliability of VA is unconvincing, the appropriate pressing force is determined based on VD1 and VD2 obtained from the DC component signal (S105). Specifically, the mean value of VD1 and VD2 may be set as the appropriate pressing force.

With this, it becomes possible to perform the appropriate pressing force determination based on both of the AC component signal and the DC component signal, and to expect improvement of precision of the determined appropriate pressing force, or the like. The processing based on both of the AC component signal and the DC component signal is not limited to the processing shown in the flowchart of Fig. 12. Other processing may be performed.

### 1-5. Display control method

The user changes the pressing force in the appropriate pressing force determination by adjusting the holding mechanism 300 (for example, adjusting the band hole position) . However, it is undesirable for the user to understand all procedures of the appropriate pressing force determination because an increased burden is imposed on the user. Therefore, in the embodiment, the pulse monitor and the user interact with each other using an interface, such as the display unit 70. A system (the pulse monitor) gives an appropriate instruction to the user to achieve reduction in burden imposed on the user.

In the appropriate pressing force determination, fluctuation in the signal value due to a factor other than change in pressing force is undesirable because determination precision is degraded. Therefore, in order to suppress the undesirable fluctuation in the signal value, determination is performed about whether or not a given condition of an environment for determination is satisfied. The appropriate pressing force determination is performed when it is determined that the condition is satisfied. When the condition is not satisfied, since some instruction for the user is required, it is desirable to use the interface such as the display unit 70.

Hereinafter, the interaction between the user and the system in the embodiment, and display control and the like which are performed at this time will be described referring to the flow of typical processing in the appropriate pressing force determination.

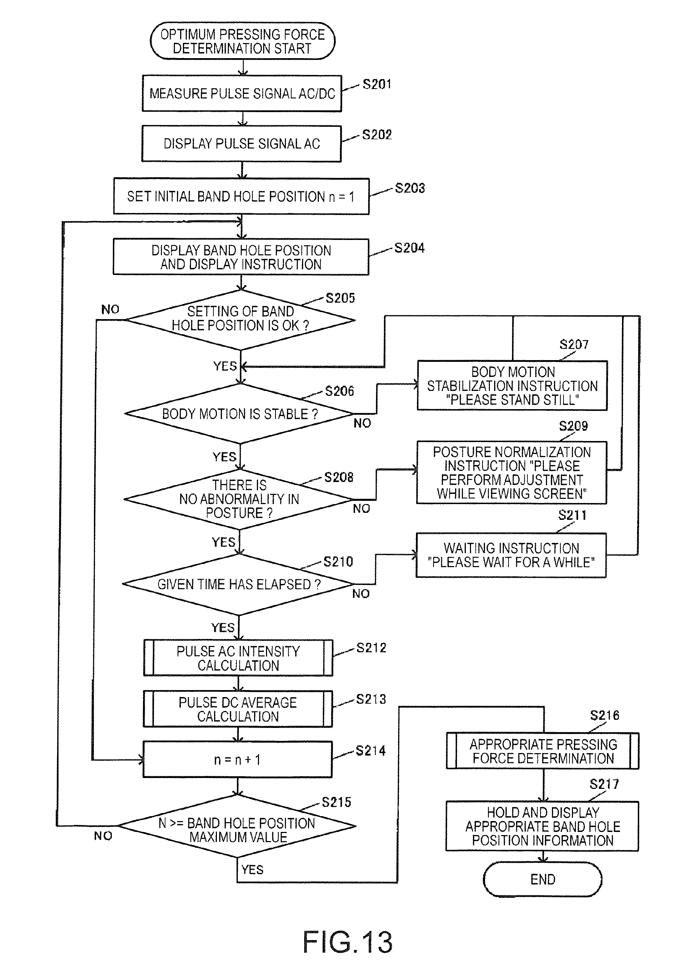

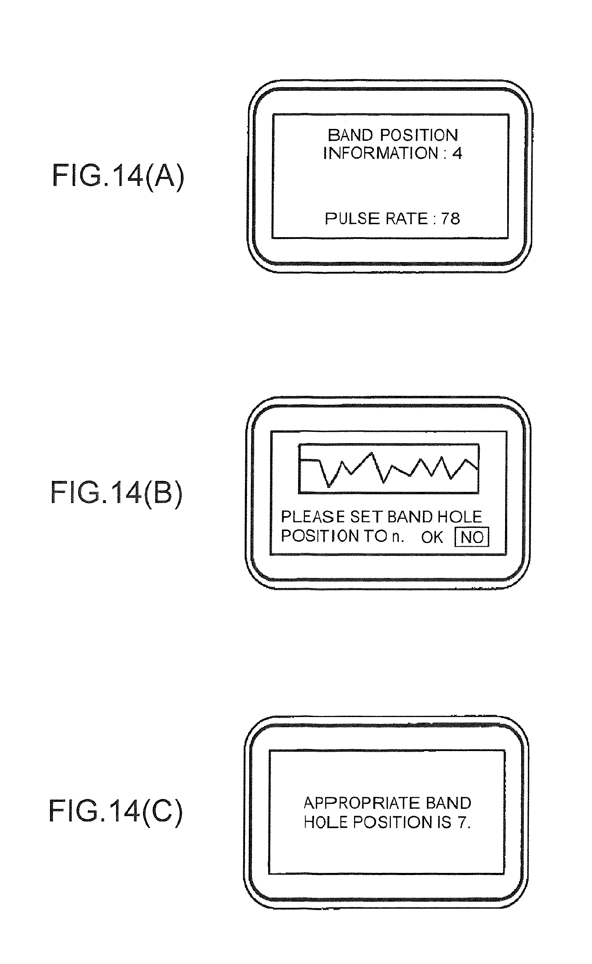

A flowchart illustrating the processing of the appropriate pressing force determination is shown in Fig. 13. When the processing starts, first, the AC component signal and the DC component signal are measured (S201). As shown on the upper side of Fig. 14(B), the waveform or the like of the AC component signal is displayed on the display unit 70 (S202). Although the display in S202 is not essential, since it is possible to give notification to the user that a signal can be appropriately acquired by the pulse wave sensor 11, it is possible to give a sense of security to the user to assure that there is no failure or the like in the device.

Thereafter, a variable n representing a band hole position is initialized (S203). Here, it is assumed that n = 1. The variable n represents a band hole position recognized by the pulse monitor side and does not ensure that the actual holding state of the holding mechanism 30 becomes the holding state by the band hole position corresponding to n. However, as described below, when the user follows an instruction on the instruction screen, the band hole position recognized by the system and the actual holding position correspond to (match) each other.

Next, as shown on the lower side of Fig. 14(B), control for displaying an instruction screen for giving an instruction to the user is performed such that the band hole position recognized on the system side and the actual holding state match each other, and an input is received from the user (S204). In the example shown in Fig. 14 (B), the user attempts to hold the pulse monitor at the instructed band hole position. If the band can be fastened, the user inputs OK. For example, if the band is too fastened and cannot be fastened or the band is too loosened and cannot be sufficiently fixed, the user inputs NG. However, the interaction with the user is not limited thereto.

The input of the user to S204 is received, and determination is performed about whether OK is input (S205). When it is determined in S205 to be Yes, it is considered that mounting at the band hole position instructed by the instruction screen is possible, and processing based on the AC component signal and the DC component signal in this holding state (and at a pressing force corresponding to the holding state) is performed. Specifically, in S206 to S211, determination is performed about whether or not the conditions of an environment for determination are satisfied. When the conditions of the environment for determination are satisfied, actual processing is performed in S212 and S213. Hereinafter, specific description will be provided.

First, as the condition of the environment for determination, determination is performed about whether or not body motion is stable (S206). This is because, when body motion is unstable, a component (body motion noise) due to body motion is included in the AC component signal and appropriate pressing force determination is hindered. The processing of S206 may be performed based on a body motion detection signal output from a body motion sensor. The body motion sensor is, for example, the acceleration sensor 21. The body motion detection signal is an acceleration detection value.

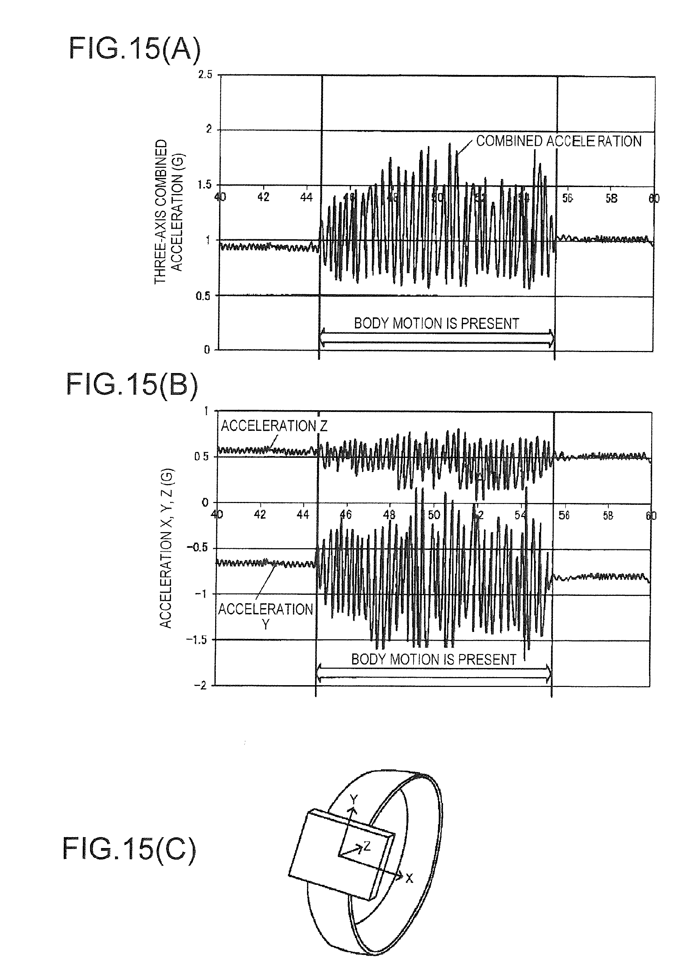

A specific example of the acceleration detection value is shown in Fig. 15(B). As will be apparent from Fig. 15(B), the magnitude of the acceleration detection value is extremely large when body motion is present compared to when body motion is absent. Therefore, it may be determined that body motion is unstable when the acceleration detection value is large and it may be determined that body motion is stable when the acceleration detection value is small.

The acceleration sensor is a three-axis acceleration sensor, and the directions of the axes thereof are as shown in Fig. 15 (C). As shown in Fig. 15 (C), when a plane including a dial portion of a wristwatch (equivalent to the display unit 70 in the pulse monitor) is considered, the 12 o'clock direction of the timepiece included in the plane is a Y axis, and the 3 o'clock direction of the timepiece is an X axis. A Z axis is an axis in a direction orthogonal to the plane including the X axis and the Y axis and directed toward the wrist side of the subject with respect to the dial portion. However, the directions of the axes are not limited thereto.

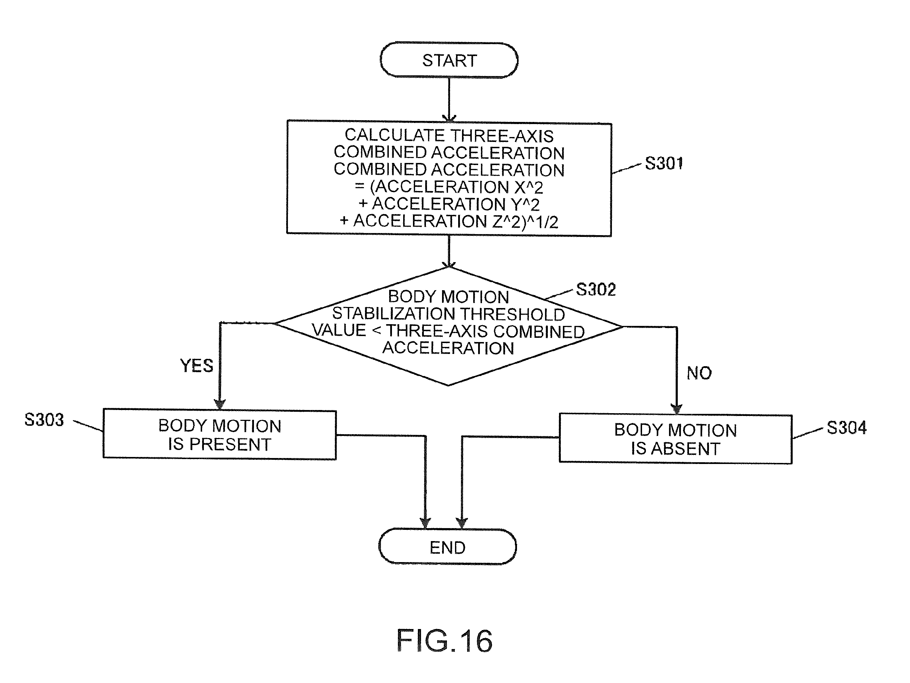

The combined acceleration of the three axes shown in Fig. 15 (A) is calculated, and determination is performed about whether body motion is stable based on comparison processing of the magnitude of the calculated combined acceleration and a given threshold value. A flowchart is shown in Fig. 16. A square root of a sum of squares of the axes is calculated as the three-axis combined acceleration (S301), and comparison processing of the calculated three-axis combined acceleration and a body motion stability threshold value is performed (S302). When the three-axis combined acceleration is greater than the body motion stability threshold value (S303), it is determined that body motion is present (body motion is unstable). Otherwise, it is determined that body motion is absent (body motion is stable) (S304).

When it is determined by the above-described processing that body motion is unstable (No in S206), it waits for body motion to stabilize. An instruction to stabilize body motion is given from the system side to the user, whereby it is considered possible to efficiently realize the condition of the environment for determination. Accordingly, control for displaying, on the display unit 70, an instruction screen for giving an instruction to stabilize body motion is performed (S207).

When it is determined in S206 to be Yes, determination is performed about whether or not there is no abnormality in the posture of the user as a second condition of the environment for determination (S208). Here, in a narrow sense, the posture indicates a posture determined by the height relationship between the mounted region (assumed to be the wrist) of the pulse wave sensor 11 and the heart. That is, since the magnitude of water head pressure applied to a blood vessel changes with change in the posture, a blood flow rate changes. The posture determination is performed as a condition for suppressing degradation in precision of the appropriate pressing force determination due to change in the blood flow rate.

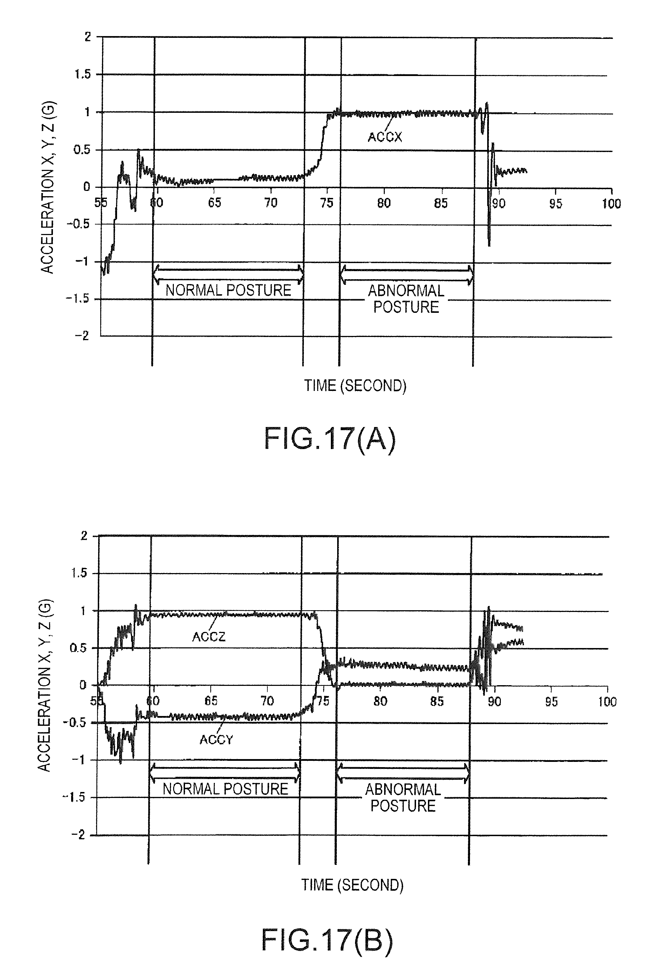

As an example, a typical posture of the user when viewing the dial portion of a wristwatch may be set as a reference posture, and a posture in which the height relationship between the wrist and the heart is significantly different may be set as an abnormal posture. An example of the acceleration detection value at this time is shown in Figs. 17 (A) and 17 (B) . Figs. 17(A) and 17(B) are merely separated due to a problem regarding graph visibility, and the values of the X axis, the Y axis, and the Z axis are acquired from the same acceleration sensor 21. Since the axes shown in Fig. 15 (C) are used as the axes of the acceleration sensor 21, the typical ranges in the reference posture of the acceleration detection values of the axes (which are primarily gravitational acceleration components acting on the axes because the acceleration detection values are based on the assumption that body motion is small) can be specified to a certain degree. For example, it is considered that the plane including the dial portion is close to the horizontal plane and the Z axis and the gravity direction are close to each other. In this case, the values of the X axis and the Y axis are close to 0 and the value of the Z axis is close to 1 G. Therefore, the appropriate ranges of the values of the axes in the reference posture are set based on these values. A posture is determined according to whether the acceleration detection values are within the appropriate ranges. As shown in Figs. 17(A) and 17(B), in the abnormal posture, the values of the respective axes are deviated from the appropriate ranges.

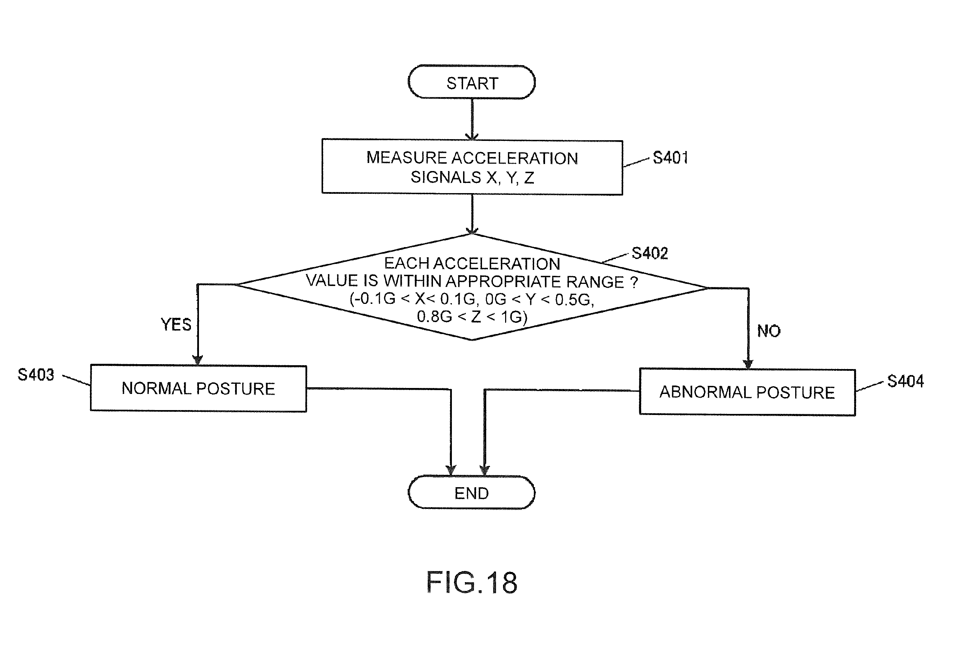

A flowchart is shown in Fig. 18. When this processing starts, first, the pulse monitor measures the acceleration detection values on the X, Y, and Z axes (S401). The pulse monitor performs comparison processing of the appropriate ranges of the axes set in advance and the acceleration detection values measured in S401 (S402). When all the acceleration detection values of the axes are within the appropriate ranges, the pulse monitor determines that the posture is a normal posture (S403). Otherwise, the pulse monitor determines that the posture is an abnormal posture (S404).

Since it is assumed that a portion above the elbow of the arm is kept in a state close to the horizontal, the appropriate range of the X axis may be a narrow range including 0 G (for example, as shown in Fig. 18, -0.1 to +0.1 G). Meanwhile, it is highly likely that the angle of the wrist is different depending on a person. It is desirable to set the appropriate ranges wider than the appropriate range of the X axis as the appropriate ranges of the Y axis and the Z axis while respectively setting 0 G and 1 G as references for the Y axis and the Z axis. The appropriate ranges are not limited to the numerical values shown in S402 in Fig. 18.

When it is determined by the above-described processing that the posture is an abnormal posture (No in S208), the pulse monitor stays on standby for the posture to be normal. At this time, as in the stabilization of body motion, the pulse monitor may perform display control for an instruction screen for giving an instruction to the user to take the normal posture (S209). In this case, taking into consideration user convenience or the like, it is desirable to display an instruction screen for clearly showing what posture is the normal posture. The pulse monitor may instruct the user to take the normal posture using a sentence shown in S209 in Fig. 13 or may instruct the user to take the normal posture by displaying a figure if there is no problem in resolution or the like of the display unit 70.

When it is determined in S208 to be Yes, as a third condition of the environment for determination, the pulse monitor determines whether or not a given time has elapsed from the mounting of the band (S210). When the holding mechanism 300 is realized by a band using a hole, or the like, during mounting at a given band hole, it is necessary to fasten the band more tightly than during mounting at the band hole once. Therefore, as shown in Fig. 9 (A) or 11, the AC component signal and the DC component signal during mounting undergo large fluctuate undesirably for the appropriate pressing force determination, and thereafter, a signal value is unstable for a given time. Therefore, in order to perform the appropriate pressing force determination, it is desirable to exclude an unstable signal value from the processing. Specifically, the pulse monitor stays on standby without performing the processing for a given time after mounting and starts the processing when the given time has elapsed.

In S210, the pulse monitor may perform determination about whether or not the given time has elapsed based on the time measurement information from the time measurement unit 140 realized by a timer or the like. When it is determined that the given time has not elapsed (No in S210), the pulse monitor performs control for displaying an instruction screen for giving an instruction to the user to stay on standby (S211).

When it is determined in S210 to be Yes, since the condition of the environment for determination is satisfied, the pulse monitor performs processing based on the AC component signal and processing based on the DC component signal (S212 and S213). In S212, processing for obtaining the amplitude value of the AC component signal is performed, and in S213, processing for obtaining a representative value (mean value) of the DC component signal is performed. The specific processing has been described above, thus, detailed description thereof will not be repeated.

After the processing of S213 or when it is determined in S205 to be No, n is incremented (S214) and determination is performed about whether or not the processing at all the band holes ends (S215). When it is determined in S215 to be No, since it is necessary to perform the processing at a band hole position equivalent to n incremented in S214, the process returns to S204, control for displaying an instruction screen for giving an instruction to the user is performed such that the band hole position recognized on the system side and the actual holding state match each other, and an input from the user is received. The same applies to subsequent processing.

When it is determined in S215 to be Yes, since the processing at all the band hole positions ends, the appropriate pressing force is determined based on the amplitude values of the AC component signal and the representative values of the DC component signal calculated at the respective band hole positions (S216). Specifically, processing shown in the flowchart of Fig. 12, or the like is performed.

A band hole position (in a broad sense, holding state specification information) corresponding to the appropriate pressing force is stored, and as shown in Fig. 14(C), control for displaying a screen for giving notification of the appropriate band hole position to the user on the display unit 70 is performed (S217).

In the embodiment, as shown in Fig. 5, the pulse monitor includes the pulse wave detection unit 10 which has the pulse wave sensor 11 configured to output a pulse wave sensor signal, the processing unit 100 which calculates pulse information based on the signal output from the pulse wave detection unit 10, the display unit 70 which displays a processing result in the processing unit 100, the storage unit 90 which stores the processing result in the processing unit 100, and the holding mechanism 300 (shown in Fig. 4(A)) which holds the pulse monitor on the subject. The processing unit 100 determines whether or not a pressing force on the subject in the pulse wave detection unit 10 is an appropriate pressing force. The storage unit 90 stores the holding state specification information when it is determined that the pressing force on the subject in the pulse wave detection unit 10 is the appropriate pressing force. The processing unit 100 performs control for displaying the holding state specification information on the display unit 70.

Here, the holding state specification information is information for specifying the holding state of the holding mechanism 300. For example, if the pulse monitor is the device held by the band as shown in Fig. 4(A), the holding state specification information is information for specifying the state of the band. Specifically, when the band is fixed using the holes as shown in Fig. 7, information indicating which band hole position is used to hold the pulse monitor is the holding state specification information. The band is not limited to the band using the holes, and a Velcro-type band having a scale or a ratchet-type band may be used. The holding mechanism 300 is not limited to the band, and an arbitrary mechanism may be used insofar as the mechanism can fix the pulse monitor to the subject and does not obstruct the acquisition of the sensor information in the pulse wave sensor 11.

With this, after performing the determination of the appropriate pressing force in the pulse monitor, it becomes possible to store and display the holding state specification information corresponding to the appropriate pressing force. Even if the appropriate pressing force can be obtained in units of kPa, mmHg, or the like, it is not easy to determine in which state the holding mechanism 300 is set to realize the appropriate pressing force. In this regard, the appropriate pressing force and the holding state specification information are associated with each other, the user side does not need to be aware of the numerical value or the like of the appropriate pressing force, and can recognize whether or not the pressing force is the appropriate pressing force by an intuitive method, such as the fastening state of the band. For this reason, after the determination of the appropriate pressing force is performed once, it is possible to easily reproduce a state in which the appropriate pressing force is applied even when the apparatus is mounted again, and to expect improvement of user convenience, or the like.

As described above, the appropriate pressing force becomes pressure within a range greater than a pressing force corresponding to a venous vanishing point (venous recovery point) and less than a pressing force corresponding to an arterial vanishing point (arterial recovery point). That is, in the embodiment, the pulse monitor may include the pulse wave detection unit 10 which has the pulse wave sensor 11 configured to output a pulse wave sensor signal, the processing unit 100 which calculates pulse information based on the signal output from the pulse wave detection unit 10, the display unit 70 which displays a processing result in the processing unit 100, the storage unit 90 which stores the processing result in the processing unit 100, and the holding mechanism 300 which holds the pulse monitor on the subject, in which the processing unit 100 may perform determination whether or not a pressing force on the subject in the pulse wave detection unit 10 falls within a range greater than a pressing force corresponding to a venous vanishing point (venous recovery point) and less than a pressing force corresponding to an arterial vanishing point (arterial recovery point), the storage unit 90 may store holding state specification information for specifying the holding state of the holding mechanism 300 when it is determined that the pressing force on the subject in the pulse wave detection unit 10 falls within the above-described range, and the processing unit 100 may perform control for displaying the holding state specification information on the display unit 70.

The processing unit 100 may perform determination about whether or not the pressing force is the appropriate pressing force based on the pulse wave sensor signal.

With this, it becomes possible to perform the appropriate pressing force determination taking an individual difference into consideration. In the method of the related art, since processing based on physical information, such as a pressure value, is performed, the individual difference is not taken into consideration. Meanwhile, the pulse wave sensor signal, which is a vital sign having a large individual difference, is used, whereby it is possible to appropriately determine the appropriate pressing force for each user. It is also possible to cope with fluctuation in appropriate pressing force based on change in physical condition or the like of the same user. In a state in which the appropriate pressing force determined by the method of the embodiment is applied, calculation of pulsation information is performed, whereby it becomes possible to achieve improvement of precision of the calculated pulse information, or the like.

The processing unit 100 may perform determination about whether or not the pressing force is the appropriate pressing force based on at least one of the AC component signal corresponding to the AC component of the pulse wave sensor signal and the DC component signal corresponding to the DC component of the pulse wave sensor signal.

Here, while the AC component signal corresponds to a high-frequency component and the DC component signal corresponds to a low-frequency component, a frequency as a reference of levels may be determined based on the frequency of a pulse, or the like. Considering the fact that the AC component signal is also used for calculation of pulse information, or the like, the AC component signal is required to include a component due to heartbeat. When the biological characteristics of the subject are considered, it is possible to set a typical lower limit value of a pulse rate to some degree, and for example, it does not often occur that the pulse rate is less than 30 per minute. Therefore, since it is considered to be rare that a signal due to heartbeat is included in a frequency band equal to or lower than a frequency (0.5 Hz) corresponding to the pulse rate of 30 per minute, a frequency higher than this frequency may be set as the high-frequency component and a frequency lower than this frequency may be set as the low-frequency component.

With this, it becomes possible to perform the appropriate pressing force determination using at least one of the AC component signal and the DC component signal. The AC component signal has a characteristic which appears in an amplitude value, and the DC component signal has a characteristic which appears at an inflection point. Although it is possible to determine the appropriate pressing force based on either the AC component signal or the DC component signal, when both signals are used, as shown in the flowchart of Fig. 12, it becomes possible to improve determination precision.

The processing unit 100 may perform determination about whether or not the pressing force is the appropriate pressing force based on the change characteristic of the AC component signal when the pressing force is changed. Specifically, the processing unit 100 may perform determination about whether or not the pressing force is the appropriate pressing force based on the change characteristic of amplitude of the AC component signal when the pressing force is changed.

With this, it becomes possible to perform determination based on the change characteristic of the AC component signal with respect to the pressing force. The pulse wave sensor signal is a vital sign having a large individual difference, and the AC component signal corresponding to the AC component of the pulse wave sensor signal also has a large individual difference. Therefore, since there is an individual difference in the magnitude of the signal value during the appropriate pressing force as well, it is difficult to perform determination about whether or not the pressing force is the appropriate pressing force only from a signal value at a given pressing force. In this regard, if the change characteristic with respect to the pressing force is used, it becomes possible to perform appropriate determination without depending on the individual difference. As shown in Fig. 8, since it is known that the AC component signal has a relatively large amplitude value at the appropriate pressing force, specifically, the determination may be performed based on the change characteristic of the amplitude value.

The processing unit 100 may perform determination about whether or not the pressing force is the appropriate pressing force based on the change characteristic of the DC component signal when the pressing force is changed. Specifically, the processing unit 100 may detect an inflection point of the DC component signal based on the change characteristic of the DC component signal when the pressing force is changed and may perform determination about whether or not the pressing force is the appropriate pressing force based on the detected inflection point.

With this, it becomes possible to perform determination based on the change characteristic of the DC component signal with respect to the pressing force. The DC component signal represents the volume of a blood flow. In a pressurization (depressurization) process, since the DC component signal has inflection points which appear at the venous vanishing point (venous recovery point) and the arterial vanishing point (arterial recovery point), it becomes possible to perform the appropriate pressing force determination with high precision, and when the appropriate pressing force is obtained within a range, it is possible to accurately determine an upper limit value and a lower limit value of the appropriate pressing force.

The processing unit 100 may perform control for displaying, on the display unit 70, an instruction screen for giving an instruction to set an environment for determination about whether or not the pressing force is the appropriate pressing force.

Here, the environment for determination means an environment which satisfies a condition that body motion is stable, a posture is not abnormal, and a given time has elapsed after the holding state of the holding mechanism 300 is determined. For the environment for determination, conditions other than the above may be added or some or all (when other conditions are added) of the conditions may be excluded. Various settings may be performed for the contents of the conditions.

With this, it becomes possible to perform the instruction to set the environment for determination. Unlike the method of the related art using a pressure value or the like, in the embodiment, since the determination is performed based on the pulse wave sensor signal which is a vital sign, a signal value fluctuates due to a factor other than change in pressing force. Since it is difficult to specify or isolate the fluctuation factor only from the signal value of the pulse wave sensor signal, it is necessary to suppress the fluctuation in the signal value due to a factor other than the pressing force as much as possible. Here, an environment satisfying this condition is set as the environment for determination, and an instruction is given to the user such that the environment for determination is satisfied, making it possible to accurately perform the appropriate pressing force determination.

The processing unit 100 may perform control for displaying, on the display unit 70, a screen for giving an instruction to stabilize body motion of the subject as the instruction screen.

With this, it becomes possible to perform an instruction to stabilize body motion. When body motion is large, a component due to body motion (body motion noise) is mixed in the pulse wave sensor signal, and body motion noise is superimposed on the AC component signal or the DC component signal calculated from the pulse wave sensor signal. Therefore, in order to suppress the influence of body motion noise and to perform accurate appropriate pressing force determination, it is desirable to achieve stabilization of body motion. Since body motion is performed by the user, and the pulse monitor is difficult to physically suppress body motion of the user, performing an instruction to the user in a simple form is a realistic and effective method.

The processing unit 100 may perform control for displaying, on the display unit 70, a screen for giving an instruction to the subject to take a given posture for determination as the instruction screen.

Here, the posture for determination indicates a posture defined based on the relative positional relationship between the heart of the subject and the pulse wave detection unit 10. If the relative positional relationship (specifically, height) between the heart of the subject and the pulse wave detection unit 10 fluctuates, a water head pressure fluctuates. For example, in a state in which the arm is lifted, a blood flow rate decreases because of change in water head pressure compared to a state in which the arm is lowered. That is, the posture for determination herein is a posture which is set to suppress the fluctuation of the blood flow rate due to the water head pressure (independently from change in pressing force change).

With this, since it is possible to suppress the abnormal posture from being taken during the appropriate pressing force determination, it becomes possible to suppress degradation in precision of the appropriate pressing force determination due to the water head pressure. In the embodiment on the assumption that the interaction on the display unit 70 is performed, although it is preferable that the posture for determination is a posture in which the user can naturally view the display unit 70 (for example, a posture when viewing the dial portion of the wristwatch), the invention is not limited thereto. As the instruction to the user, as in the stabilization of body motion, a method of displaying the instruction on the display unit 70 is considered simple and effective.

The processing unit 100 may perform control for displaying, on the display unit 70, a screen for giving an instruction to the subject to stay on standby for a given time as the instruction screen.

With this, the user stays on standby for the given time, whereby it becomes possible to accurately perform the appropriate pressing force determination. This is because a certain time is required when band adjustment is actually performed manually or a given time (about 10 seconds) is required until the signal value itself is stabilized as shown in Figs. 9(A) and 11.

The processing unit 100 may perform determination about whether or not the condition of the environment for determination displayed on the instruction screen is satisfied, and may perform determination about whether or not the pressing force is the appropriate pressing force when it is determined that the condition is satisfied. Specifically, the processing unit 100 performs determination about whether or not the condition for the environment for determination displayed on the instruction screen is satisfied based on a body motion detection signal output from the body motion sensor or time measurement information output from the time measurement unit.

With this, when the condition for the environment for determination is satisfied, it becomes possible to perform the appropriate pressing force determination. The instruction screen is displayed on the display unit 70, whereby it is possible to send an instruction to realize the environment for determination to the user. However, it is not ensured whether or not the user actually follows the instruction. Therefore, determination is performed on the system side about whether or not the condition is satisfied and, when the condition is satisfied, the processing is performed, whereby it becomes possible to achieve improvement of determination precision, or the like. For the stabilization of body motion and the exclusion of the abnormal posture, the body motion sensor, such as the acceleration sensor 21, may be used as shown in Figs. 15 (A), 17(A), and 17(B). In regard to the lapse of time, time measurement information output from the time measurement unit 140, which is realized by a timer or the like, may be used.

The processing unit 100 may have a holding state specification information acquisition mode and a pulse information calculation mode, in which pulse information is calculated, as a processing mode. When the holding state specification information acquisition mode is set, holding state specification information is acquired based on a determination result about whether or not the pressing force is the appropriate pressing force and the acquired holding state specification information is stored in the storage unit 90. When the pulsation information calculation mode is set after the acquisition of the holding state specification information, the processing unit 100 may perform control for reading the holding state specification information stored in the storage unit 90 and displaying the read holding state specification information on the display unit 70.

With this, after the appropriate pressing force determination is performed and the holding state specification information is acquired in the holding state specification information acquisition mode, it is possible to display the acquired holding state specification information on the display unit 70 in the pulse information calculation mode. For this reason, it becomes possible to easily realize a holding state suitable for calculation at the time of calculation of pulse information. Even if the appropriate pressing force is determined and the holding state specification information at the appropriate pressing force is calculated, it is ineffective if the result cannot be used for the calculation of the pulse information, which is main processing of the pulse monitor. Therefore, considering the fact that the acquisition of the holding state specification information and the calculation of the pulse information are not continuous, or the like, it is necessary to store the acquired holding state specification information in the storage unit 90 once. If the pulse information calculation mode is entered, it is necessary to present the holding state specification information to the user in a simple form.

The holding mechanism 300 may take first to N-th (where N is an integer equal to or greater than 2) states, in which the pressing force on the subject in the pulse wave detection unit 10 is different, as the holding state. The processing unit 100 acquires information corresponding to at least one of the first to N-th states as the holding state specification information based on a determination result about whether or not the pressing force in at least each of the first to N-th states is the appropriate pressing force.

With this, in the holding mechanism 300 (for example, a band including holes) which can take a plurality of discrete holding states, it becomes possible to perform the processing and acquire at least one state (for example, a state of being held at any band hole position) among the plurality of holding states as the holding state specification information of the result.

After determination about whether or not a pressing force in an i-th (where i is an integer which satisfies 1 ≤ i ≤ N) state is an appropriate pressing force, the processing unit 100 may perform control for displaying, on the display unit 70, an instruction screen for giving an instruction to change the holding state of the holding mechanism 300 to a j-th (where j is an integer which satisfies 1 ≤ j ≤ N and j ≠ i) state, in which a pressing force is less than the pressing force in the i-th state.

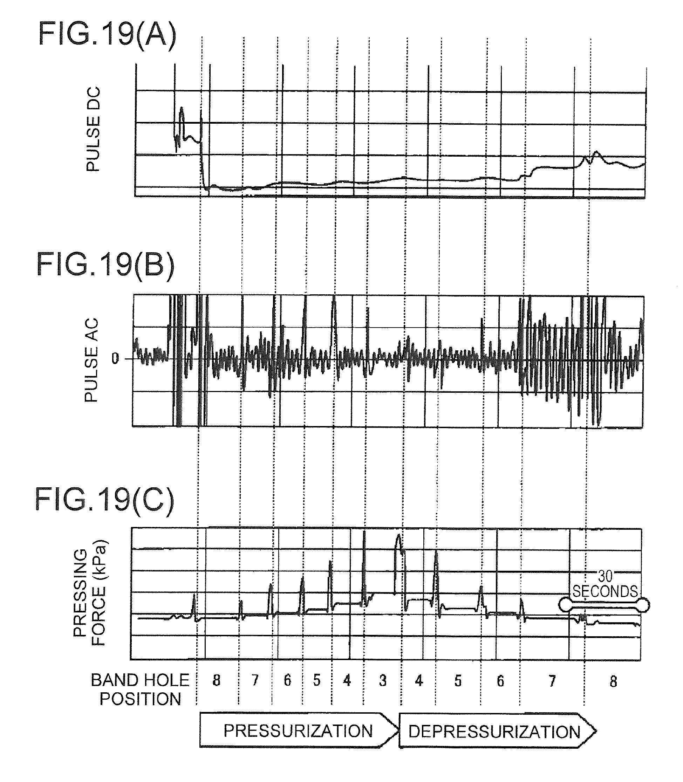

With this, it becomes possible to perform the appropriate pressing force determination in the depressurization process. An example is shown in Figs. 19(A) to 19(C). As shown in Fig. 19(C), pressurization is performed at the first half and depressurization is performed at the second half. Although an extremely large pressing force appears during changing of a band hole position, and a large signal value appears in the AC component signal corresponding to the pressing force, as shown in Fig. 9(A) and the like, the pressing force and the signal value are excluded in the appropriate pressing force determination.

As shown in Fig. 19 (B), in the AC component signal, since large amplitude appears before and after a band hole position 7 in both of the pressurization process and the depressurization process, the determination of the appropriate pressing force is possible. However, since an amplitude value is greater in the depressurization process, the determination is considered easy.

As shown in Fig. 19(A), whereas an inflection point of the DC component signal is clear in the depressurization process, the inflection point is unclear in the pressurization process. If even small fluctuation in a signal value is taken into consideration, whereas it is possible to detect the inflection point of the DC component signal in the pressurization process, the determination is easy in the depressurization process as in the AC component signal.

From the above, the appropriate pressing force determination is performed in the depressurization process, whereby it becomes possible to accurately perform the determination compared to the determination using the pressurization process.

The embodiment can be applied to a pulse monitor including the pulse wave detection unit 10 which has the pulse wave sensor 11, the processing unit 100 which performs determination about whether or not a pressing force on a subject in the pulse wave detection unit 10 is an appropriate pressing force and calculates pulse information of the subject based on a signal output from the pulse wave detection unit 10, the display unit 70 which displays a processing result in the processing unit 100, and the storage unit 90 which stores the processing result in the processing unit 100. The processing unit 100 performs control for displaying, on the display unit 70, an instruction screen for giving an instruction to set an environment for determination about whether or not the pressing force is the appropriate pressing force.

With this, even when the pressing force is changed by a method other than the holding by the holding mechanism 300, the instruction screen of the environment for determination is displayed, whereby it becomes possible to perform the pressing force determination in an appropriate situation. Since it is undesirable that a signal value for use in the appropriate pressing force determination fluctuates due to a factor other than change in pressing force regardless of a method of changing a pressing force, the setting of the environment for determination is important, and there is a significant advantage in displaying, on the display unit 70, the instruction screen for giving the instruction to set the environment for determination.

The processing unit 100 may perform determination about whether or not the condition of the environment for determination displayed on the instruction screen is satisfied and may perform determination about whether or not the pressing force is the appropriate pressing force based on the signal output from the pulse wave detection unit 10 when it is determined when the condition is satisfied.

With this, it becomes possible to perform the appropriate pressing force determination based on a signal (in a narrow sense, a pulse wave sensor signal, and in a narrower sense, at least one of an AC component signal and a DC component signal) output when the condition of the environment for determination is satisfied. Advantages for the determination on the system side about whether the condition of the environment for determination is satisfied and the use of the signal output from the pulse wave detection unit 10 in the appropriate pressing force determination are as described above.

In the pulse monitor of the embodiment, a part or the whole of the processing may be realized by a program. In this case, a processor, such as a CPU, executes the program, whereby the pulse monitor of the embodiment is realized. Specifically, the program stored in an information storage medium is read, and the read program is executed by the processor, such as a CPU. Here, the information storage medium (computer-readable medium) stores a program, data, and the like, and the function thereof can be realized by an optical disc (DVD, CD, or the like), an HDD (hard disk drive), a memory (card-type memory, ROM, or the like), or the like. The processor, such as a CPU, performs various kinds of processing of the embodiment based on the program (data) stored in the information storage medium. That is, the information storage medium stores a program which causes a computer (a device including an operating unit, a processing unit, a storage unit, and an output unit) to function as the respective units of the embodiment (a program which causes a computer to execute the processing of the respective units).

### (Second Embodiment)

### 2-1. Outline of method of the embodiment

When performing body motion noise removal (reduction), whereas a body motion detection signal is obtained from a signal acquired from a pressure sensor, a motion sensor, or the like and handled as a body motion noise component, noise may also be mixed in the body motion detection signal obtained at this time. When removing (reducing) the body motion noise component from the pulse wave sensor signal using the body motion detection signal including noise, pulse information to be detected is influenced by noise, and it is likely that the value of the pulse information does not accurately represent the actual pulse of the person with the apparatus mounted as when the body motion nose component is not removed (reduced).

When obtaining a body motion detection signal using a contact pressure sensor in PTL 1 described above, a pulse component may be included in the body motion detection signal (contact pressure detection signal) depending on the magnitude of contact pressure of a contact pressure sensor and a living body (a region to be measured). This will be apparent from the measurement principle of an oscillometric method, which is one of indirect blood pressure measurement methods.

When performing body motion noise reduction processing using a body motion detection signal including a pulse component as a body motion noise component, not only an actual body motion noise component but also a pulse component which will be detected normally may be attenuated.

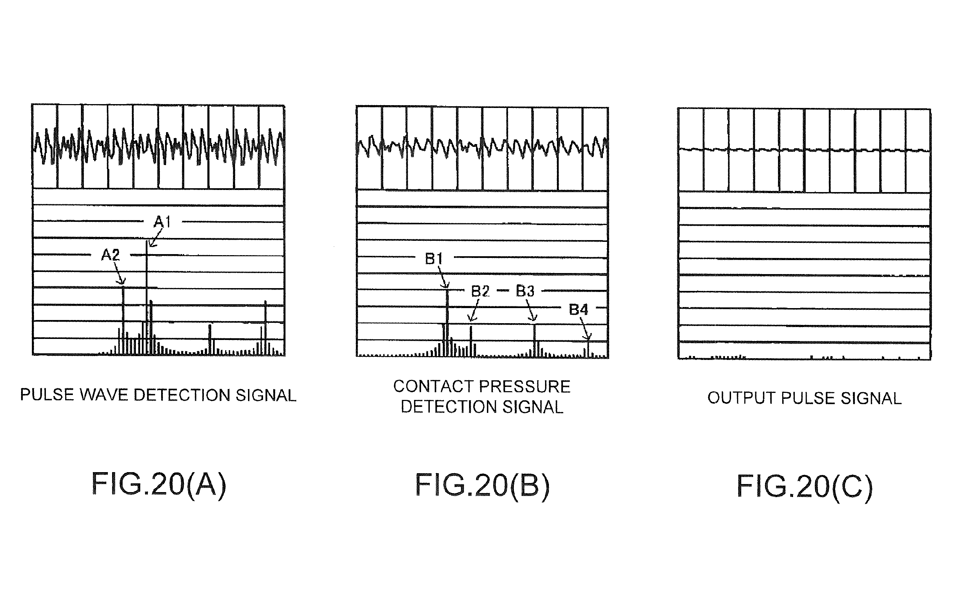

Specifically, there are cases shown in Figs. 20(A) to 20(C). A pulse wave detection signal shown in Fig. 20 (A) includes two fundamental frequencies of A1 and A2, one of the two fundamental frequencies has a pulse as a signal source, and the other one has body motion as a signal source. A contact pressure detection signal shown in Fig. 20(B) also includes two fundamental frequencies of B1 and B2, one of the two fundamental frequencies has a pulse as a signal source, and the other one has body motion as a signal source. As shown in Fig. 20(C), if body motion noise reduction processing is performed for the pulse wave detection signal of Fig. 20(A) using the contact pressure detection signal of Fig. 20(B), not only a body motion noise component but also a pulse component which will be detected normally are attenuated, and an output pulse signal having amplitude effective for obtaining accurate pulse information is not obtained.

For this reason, if the contact pressure sensor is mounted while adjusting a pressing force such that the contact pressure sensor does not detect a pulse, there is no problem. Meanwhile, actually, it is difficult to adjust a pressing force when the contact pressure sensor is mounted on the user, and convenience is significantly damaged. Normally, for example, even when the contact pressure sensor is mounted at a pressing force enough to detect a pulse, it is preferable to obtain pulse information without remounting sensors.

Accordingly, even when a pulse component is included in a body motion detection signal, a pulse detector of the embodiment or the like performs appropriate body motion noise reduction processing. Specifically, the pulse detector of the embodiment or the like reduces a body motion noise component included in a pulse wave detection signal using a body motion detection signal which is determined to include no pulse component. Hereinafter, a system configuration example and details of processing will be described in order.

### 2-2. System configuration example

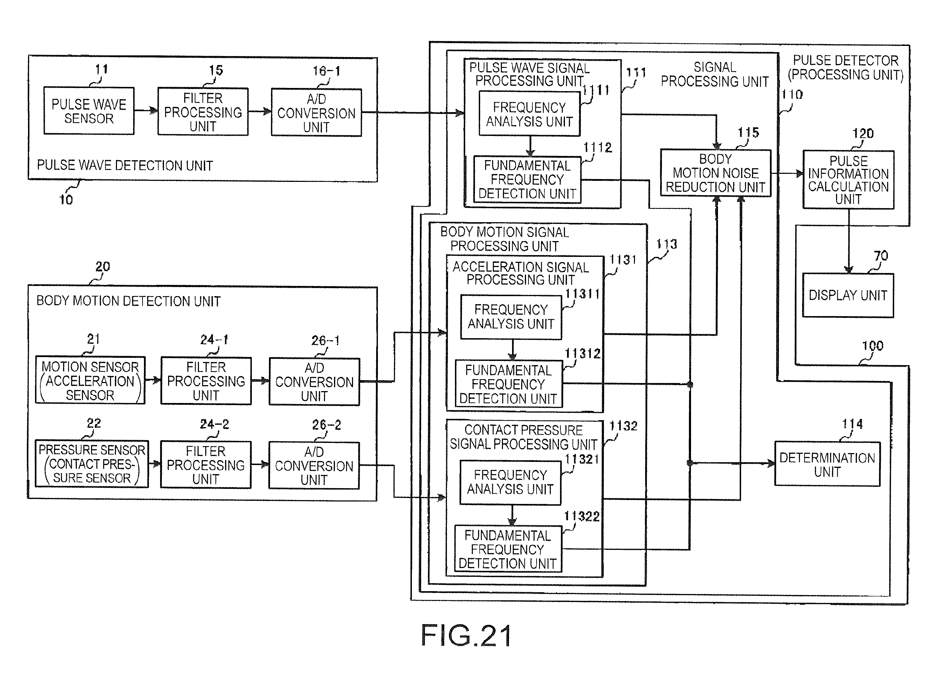

First, Fig. 21 shows a configuration example of a pulse detector (processing unit) 100 of the embodiment and an electronic apparatus including the same.

The pulse detector (processing unit) 100 includes a signal processing unit 110 and a pulse information calculation unit 120. An example of the electronic apparatus including the pulse detector 100 is a pulse monitor, a pedometer, or the like including the pulse detector 100, the pulse wave detection unit 10, the body motion detection unit 20, the display unit 70, and the like. The pulse detector 100 and the electronic apparatus including the same are not limited to the configuration of Fig. 21, and various modification may be made, in which some constituent elements are omitted, other constituent elements are added, or the like. Some or all of the functions of the pulse detector 100 may be realized by the electronic apparatus. Some or all of the functions of the pulse detector 100 of the embodiment may be realized by a server which is connected to the pulse detector 100 by communication.

Next, processing which is performed by the respective units of the pulse detector 100 will be described.

First, the signal processing unit 110 performs various kinds of signal processing described below for a pulse wave detection signal from the pulse wave detection unit 10 and a body motion detection signal from the body motion detection unit 20. The signal processing unit 110 includes a pulse wave signal processing unit 111, a body motion signal processing unit 113, a determination unit 114, and a body motion noise reduction unit 115.

The pulse wave signal processing unit 111 performs signal processing for a pulse wave detection signal from the pulse wave detection unit 10. Specifically, the pulse wave signal processing unit 111 can include a frequency analysis unit 1111 and a fundamental frequency detection unit 1112.

The frequency analysis unit 1111 performs frequency spectrum analysis processing (frequency decomposition processing) for the pulse wave detection signal. The frequency spectrum analysis processing is processing for quantitatively obtaining intensity of each frequency, and is performed for a short time region of a signal, for a long-term region, or for various functions. Specifically, the processing refers to processing, such as FFT.

The fundamental frequency detection unit 1112 performs fundamental frequency detection processing for the pulse wave detection signal based on the result of the frequency spectrum analysis processing of the frequency analysis unit 1111.

The body motion signal processing unit 113 performs signal processing for a body motion detection signal from the body motion detection unit 20. Specifically, the body motion signal processing unit 113 can include an acceleration signal processing unit 1131 and a contact pressure signal processing unit 1132.

The acceleration signal processing unit 1131 performs various kinds of signal processing with an acceleration detection signal as the body motion detection signal. Specifically, the acceleration signal processing unit 1131 can include a frequency analysis unit 11311 and a fundamental frequency detection unit 11312.

The contact pressure signal processing unit 1132 performs various kinds of signal processing with a contact pressure detection signal as the body motion detection signal. Specifically, the contact pressure signal processing unit 1132 can include a frequency analysis unit 11321 and a fundamental frequency detection unit 11322.

The functions of the frequency analysis unit 11311 and the frequency analysis unit 11321 are the same as the function of the frequency analysis unit 1111, and the functions of the fundamental frequency detection unit 11312 and the fundamental frequency detection unit 11322 are the same as the function of the fundamental frequency detection unit 1112, thus, description thereof will not be repeated.

The determination unit 114 performs determination processing of a body motion detection signal for use in body motion noise reduction processing. For example, as described below, determination is performed about which of the acceleration detection signal or the contact pressure detection signal is used as the body motion detection signal.

The body motion noise reduction unit 115 performs body motion noise reduction processing for the pulse wave detection signal from the pulse wave signal processing unit 111 based on the body motion detection signal from the body motion signal processing unit 113. The processing contents of the body motion noise reduction unit 115 and the configuration of the pulse information calculation unit 120, the pulse wave detection unit 10, the body motion detection unit 20, and the display unit 70 are the same as those shown in Fig. 1, thus detailed description thereof will not be repeated.

The functions of the respective units in the signal processing unit 110 and the signal processing unit 110, and the pulse information calculation unit 120 can be realized by hardware, such as various processors (CPU and the like) or ASIC (gate array or the like), a program, or the like. The signal processing unit 110, the pulse wave signal processing unit 111, the body motion signal processing unit 113, the acceleration signal processing unit 1131, and the contact pressure signal processing unit 1132 are not limited to the configuration of Fig. 21, and various modifications may be made, in which some constituent elements are omitted, other constituent elements are added, or the like.

### 2-3. Details of processing of the embodiment

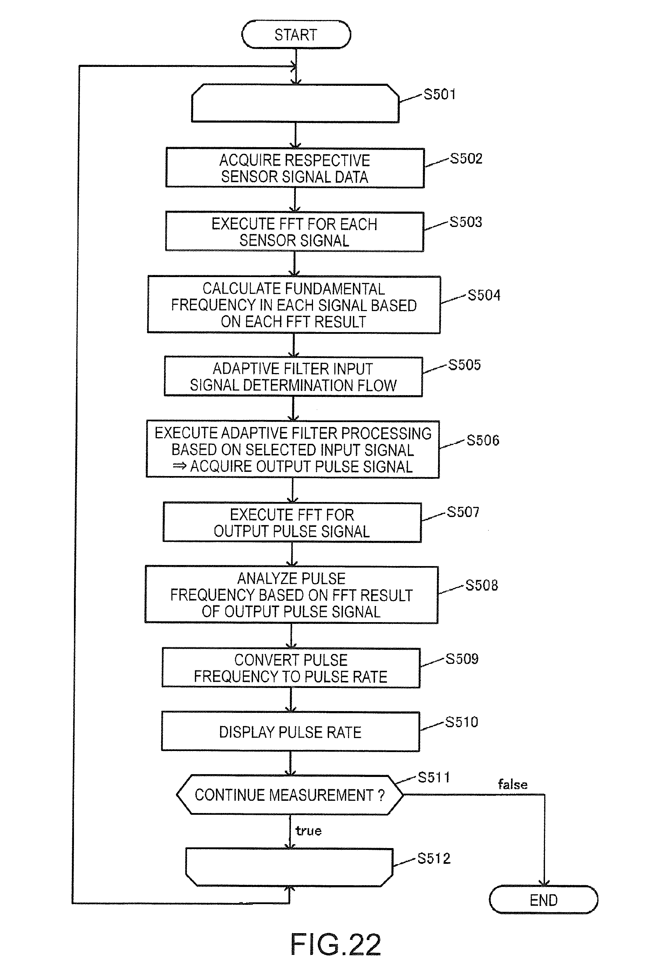

First, the entire flow of processing which is performed by the pulse detector of the embodiment or the like will be described referring to the flowchart of Fig. 22.

At the beginning, loop processing starts (S501), and various kinds of sensor signal data are acquired (S502). Since the contents of signal data acquired from various sensors are different before and after respective signal processing, signal data is distinguished by separate names. For this reason, various terms will be explained once.

First, the term "pulse" refers to motion when a peripheral blood vessel dilates or constricts. A pulse wave expresses, as a signal, change in volume caused by the flow of blood into a body tissue. For example, a pulse wave expresses, as a signal waveform, scattered light, reflected light, or the like extracted by a photodiode when LED is irradiated from a pulse wave sensor onto a body surface. A pulse wave expresses a blood vessel movement response, not the motion of the heart, and includes change in volume of a blood vessel or the like caused by a noise factor other than the motion of the heart, for example, human movement, operation, or the like. In order to correctly express the motion of the heat or the pulse rate, it is necessary to remove the noise factor.

Although, in medicine, a pulse indicates movement which occurs when periodic constriction and relaxation of internal organs as well as the heart are repeated, in this case, in particular, the motion of the heart as a pump for sending blood periodically is called a pulse.

A body motion means, in a broad sense, all cases where the body is moved, and indicates, in a narrow sense, normal and periodic motion of an arm (near a region where the pulse monitor is mounted) or the like accompanied by walking, jogging, or the like.

The term "pulse wave sensor signal (pulse wave sensor source signal, pulse wave signal)" refers to a signal which is detected by the pulse wave sensor 11. The pulse wave sensor signal includes a pulse component signal, a body motion noise component signal, a disturbance noise component signal, and the like. Whereas it is considered that an aperiodic pulse is strictly included in a pulse component signal, it is assumed that the aperiodic pulse is included in a disturbance noise component signal as an electrical signal. A pulse wave sensor source signal means a signal output from the pulse wave sensor, and is intended to emphasize the fact that the signal is not subjected to any filter processing.

The term "pulse component (pulse component signal)" refers to a component signal, which represents change in volume of a blood vessel due to the pulse of the heart, or the like, among the component signals included in the pulse wave sensor signal. In many cases, the pulse component is a periodic signal.

The term "body motion noise component (body motion noise component signal)" refers to a component signal, which represents change in volume of a blood vessel due to normal human movement, operation (body motion), or the like, among the component signals included in the pulse wave sensor signal. For example, in a pulse monitor mounted on an arm or a finger, change in volume occurs in a blood vessel by the influence of arm swing during walking, jogging, or the like. In this way, when a human performs normal operation, the body motion noise component becomes a periodic signal and a component signal having the frequency of the operation. The body motion noise component has a feature that there is high correlation with the waveform of a signal output from the acceleration sensor mounted near the pulse wave sensor mounting region.

The term "disturbance noise component (disturbance noise component signal)" refers to a component signal, which represents change in volume of a blood vessel due to moving or hitting of a region (for example, finger, hand, arm, or the like) around the pulse wave sensor, separately from the body motion noise component. For this reason, in many cases, the disturbance noise component becomes an aperiodic signal.

The term "pulse wave detection signal" refers to a signal which is output from the pulse wave detection unit 10. Specifically, the term "pulse wave detection signal" refers to a signal after the filter processing unit 15 performs filter processing for the pulse wave sensor signal and the A/D conversion unit 16-1 performs A/D conversion processing for the pulse wave sensor signal after the filter processing, or the like. However, as described above, the order of the filter processing and A/D conversion processing may be reversed.

Next, the term "body motion sensor signal (body motion sensor source signal, body motion signal)" refers to a signal which is detected by the body motion sensor. Specifically, the term "body motion sensor signal" indicates a motion sensor signal which indicates a signal to be detected by a motion sensor (acceleration sensor) 21, a contact pressure sensor signal which indicates a signal to be detected by a contact pressure sensor (pressure sensor) 22, or the like.

The motion sensor 21 is, for example, an acceleration sensor 21. The acceleration sensor 21 has, for example, an element in which a resistance value increases or decreases depending on an external force, and detects acceleration information of the three axes.

The pressure sensor 22 is, for example, the contact pressure sensor 22. The contact pressure sensor 22 may come into direct contact with the subject to measure a contact pressure, or may indirectly measure a contact pressure by a cuff structure or the like. That is, a sensor using a piezoelectric element may be used, or an atmospheric pressure sensor or the like may be used.

As described referring to Figs. 20(A) to 20(C), as in the pulse wave sensor signal, a pulse component may also be included in the contact pressure sensor signal, and body motion noise reduction processing at this time is the subject of the embodiment. A possibility that a pulse component is included in the motion sensor signal, such as the acceleration sensor signal, is extremely small, and even if the pulse component is included, the pulse component is negligibly small. However, whereas the contact pressure sensor can detect body motion, such as clenching of hands, the motion sensor 21 cannot detect body motion not enough to cause change in the position of each part of the subject. Accordingly, in the embodiment, as described below, body motion noise reduction processing is performed using the contact pressure sensor preferentially.

The term "body motion detection signal" refers to a signal which is output from the body motion detection unit 20. Specifically, the term "body motion detection signal" refers to a signal after filter processing and A/D conversion processing are performed for the body motion sensor signal, or the like. for example, the body motion detection signal is a motion detection signal which is a signal after the above-described signal processing is performed for the motion sensor signal, a pressure detection signal which is a signal after the above-described signal processing is performed for the pressure sensor signal. However, as described above, the order of the filter processing and the A/D conversion processing may be reversed.

As described above, in Step S502, the pulse wave signal processing unit 111 acquires the pulse wave detection signal, and the body motion signal processing unit 113 acquires the body motion detection signal.

Next, the pulse wave signal processing unit 111 performs FFT for the pulse wave detection signal for a predetermined period (for example, 16 seconds), and the body motion signal processing unit 113 performs FFT for the body motion detection signal for a predetermined period (for example, 16 seconds) (S503). The pulse wave signal processing unit 111 and the body motion signal processing unit 113 calculate the fundamental frequencies included in the respective signals based on the results of FFT (S504).

The determination unit 114 performs determination processing of an adaptive filter input signal based on the calculated fundamental frequencies and the like (S505). The processing of Step S505 will be described below in detail.

Next, the body motion noise reduction unit 115 executes as body motion noise reduction processing, adaptive filter processing based on the input signal selected by the determination unit 114, acquires an output pulse signal, and outputs the output pulse signal to the pulse information calculation unit 120 (S506).

Thereafter, the pulse information calculation unit 120 performs FFT for the output pulse signal (S507), and analyzes a pulse frequency based on the FFT result (S508). The pulse frequency is converted to a pulse rate (S509), and the pulse rate is displayed on the display unit 70 (S510).

Finally, determination is performed about whether or not to continue measurement (S511), and when it is determined to continue measurement, the processing from Step S501 to Step S512 is repeated. When it is determined to end measurement, the entire processing ends.

As shown in Fig. 21, the pulse detector 100 of the embodiment described above includes the body motion noise reduction unit 115 which performs the body motion noise reduction processing for reducing the body motion noise component included in the pulse wave detection signal from the pulse wave detection unit 10 having the pulse wave sensor 11, and the determination unit 114 which performs the determination processing of the body motion detection signal for use in the body motion noise reduction processing. The determination unit 114 performs determination about whether or not a pulse component is included in the contact pressure detection signal from the body motion detection unit 20 having the contact pressure sensor 22 and performs the determination processing about whether or not to use the contact pressure detection signal as the body motion detection signal. The body motion noise reduction unit 115 performs the body motion noise reduction processing based on the pulse wave detection signal and the contact pressure detection signal when it is determined to use the contact pressure detection signal as the body motion detection signal.

In this way, the pulse detector 100 of the embodiment can perform determination about whether or not the pulse component is included in the contact pressure detection signal, and can perform determination about whether or not to actually use the contact pressure detection signal as the body motion detection signal in the body motion noise reduction processing.

For this reason, it becomes possible to use the contact pressure detection signal insofar as the contact pressure detection signal is effectively used in the body motion noise reduction processing, and even when the pulse component is included in the body motion detection signal, it is possible to perform appropriate body motion noise reduction processing.

With this, for example, when the user mounts sensors on the body, it is not necessary to mount the sensor while adjusting the pressing force, and it becomes possible to provide a pulse detector having high user convenience.

As the determination processing of the body motion detection signal for use in the body motion noise reduction processing, instead of the above-described processing for determining whether or not to use the contact pressure detection signal as the body motion detection signal, processing described below may be performed. The body motion noise reduction processing is explained as described above referring to Figs. 2 and 3(A) to 3(C).

The determination unit 114 may perform the determination processing to use the contact pressure detection signal as the body motion detection signal when it is determined that no pulse component is included in the contact pressure detection signal. Alternatively, the determination unit 114 may perform the determination processing not to use the contact pressure detection signal as the body motion detection signal when it is determined that the pulse component is included in the contact pressure detection signal.

Accordingly, when the pulse component is included in the contact pressure detection signal, since the contact pressure detection signal cannot be used in the body motion noise reduction processing, there is no concern that the pulse component included in the pulse wave detection signal is attenuated. For this reason, the contact pressure detection signal can be used positively in the body motion noise reduction processing. As described above, whereas the contact pressure sensor can detect body motion, such as clenching of hands, the motion sensor cannot detect body motion not enough to cause change in the position of each part of the subject. According to the embodiment, even when the body motion noise component, such as clenching of hands, which cannot be detected by the motion sensor is included in the pulse wave detection signal, it becomes possible to reduce or remove the body motion noise component. For this reason, it becomes possible to accurately obtain pulse information compared to a case where the body motion noise reduction processing is performed using the motion detection signal.

However, even when the pulse component is included in the contact pressure detection signal, it is necessary to obtain pulse information. In this case, the contact pressure detection signal may be used. As described above, a possibility that the pulse component is included in the motion sensor signal, such as the acceleration sensor signal, is extremely small, and even if the pulse component is included, the pulse component is negligibly small. As described above, although there is body motion which cannot be detected by the motion sensor, it is preferable to obtain pulse information while precision is somewhat degraded compared to all pieces of pulse information are not obtained.

Accordingly, the body motion detection unit 20 may have a motion sensor. The determination unit 114 may perform determination processing to use a motion detection signal from the body motion detection unit 20 as the body motion detection signal when it is determined that the pulse component is included in the contact pressure detection signal.

With this, it becomes possible to perform the body motion noise reduction processing using the motion detection signal and to obtain the pulse information even when the pulse component is included in the contact pressure detection signal.

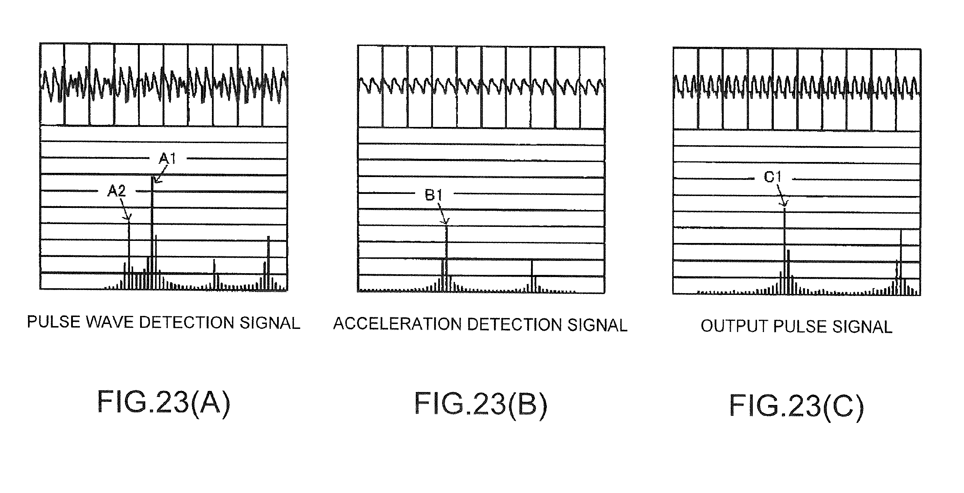

As a specific example, Figs. 23(A) to 23(C) show results when body motion noise reduction processing is performed using an acceleration detection signal. Whereas two fundamental frequencies (A1 and A2) representing pulse and body motion are included in the pulse wave detection signal, only one fundamental frequency B1 representing body motion is included in the acceleration detection signal. For this reason, if the body motion noise reduction processing is performed using the contact pressure detection signal, even in the cases of Figs. 20 (A) to 20(C), as shown in Fig. 23 (C), it is possible to obtain an output pulse signal having amplitude effective for obtaining pulse information.

Next, a specific method of performing determination about whether or not a pulse component is included in a contact pressure detection signal will be described. As described above, the pulse is periodic motion, and body motion which is handled in the embodiment is periodic motion. That is, since signals representing pulse and body motion are periodic signals, in order to perform determination about whether or not the pulse component is included in the contact pressure detection signal, it is preferable to know how many periodic signals are included in the contact pressure detection signal.

Accordingly, the pulse detector 100 of the embodiment may include the body motion signal processing unit 113 which performs the detection processing of the fundamental frequency of the contact pressure detection signal. The determination unit 114 may perform the determination processing based on the detection processing result of the fundamental frequency of the contact pressure detection signal. That is, the determination unit 114 may perform determination about whether or not the pulse component is included in the contact pressure detection signal based on the detection processing result of the fundamental frequency, and may perform determination about whether or not to use the contact pressure detection signal in the body motion noise reduction processing based on the determination result.

A fundamental frequency indicates a frequency of a lowest frequency component when a signal is represented by sine wave synthesis (for example, Fourier series). In other words, the fundamental frequency refers to a repetition frequency of a minimum period section of a periodic signal and is associated with a signal source of the periodic signal on a one-to-one basis. That is, if it is known how many fundamental frequencies are included in a signal, such as the contact pressure detection signal, it is possible to specify how many signal sources of the periodic signal are provided. Meanwhile, a frequency component which is an integer multiple of the fundamental frequency is referred to as harmonic. Specifically, in Fig. 20(B), B1 and B2 are fundamental frequencies for separate signal sources, B3 is a harmonic of B1, and B4 is a harmonic of B2. Since a signal source of a periodic signal which can be observed during periodic movement, such as running, is body motion or a pulse, one of B1 and B2 is a body motion component, and the other one is a pulse component.

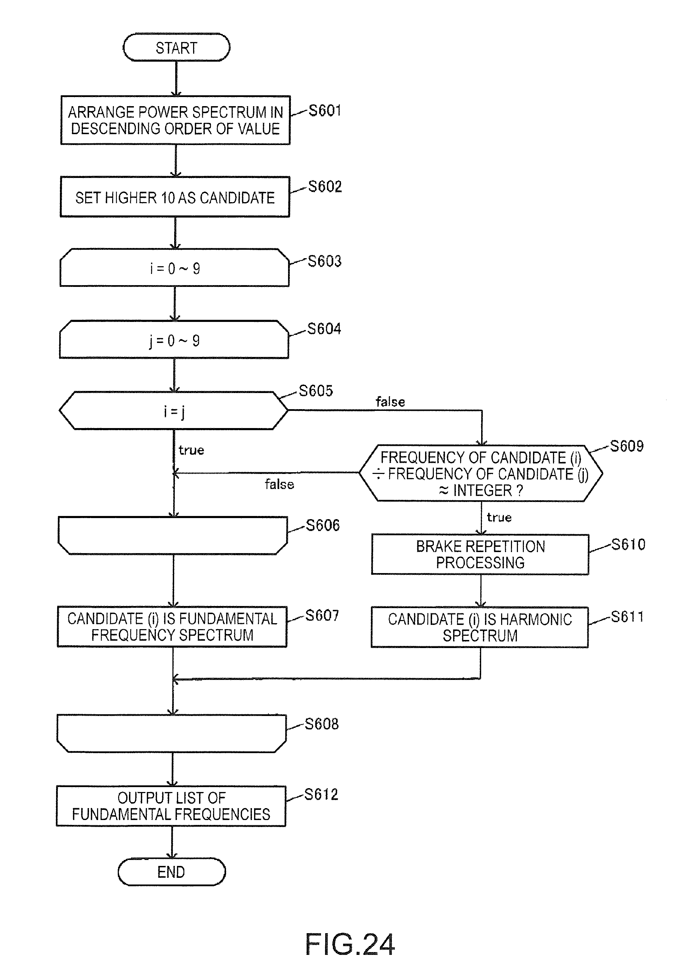

As a specific example, the flow of fundamental frequency calculation processing will be described referring to the flowchart of Fig. 24. This processing is processing which is performed in Step S504 of Fig. 22, or the like.

In the processing shown in Fig. 24, ten power spectrums of the contact pressure detection signal are selected in an ascending order, the selected ten power spectrums are round-robin compared, if a frequency of a certain power spectrum is an integer multiple of a frequency of a different power spectrum, it is determined that the power spectrum is a harmonic spectrum, and if a frequency of a certain power spectrum is not an integer multiple of a frequency of any power spectrum, it is determined that the power spectrum is a fundamental frequency spectrum.

Hereinafter, description will be provided in order. First, the power spectrums of the contact pressure detection signal are rearranged in a descending order (S601), and ten higher power spectrums in an ascending order are set as candidates of a fundamental frequency spectrum (S602). The number of power spectrums as candidates is not necessarily limited to ten, and for example, a predetermined threshold value for a power spectrums may be provided, and ten power spectrums which are equal to or greater than the threshold value in an ascending order may be set as candidates, or the like. In this case, if there is no power spectrum which exceeds the threshold value, it may be determined to be "no candidate", and in this case, the body motion noise reduction processing may be performed using the motion detection signal, or the like.

Next, first loop processing from Step S603 to Step S608 is repeated while changing i in a range of i = 0 to 9.

In the first loop, second loop processing from Step S604 to Step S606 is repeated while changing j in a range of j = 0 to 9.

In the second loop processing, determination is performed about whether or not i and j are the same (S605), and when i and j are different, determination is performed about whether or not a result obtained by dividing a frequency of an i-th candidate by a frequency of a j-th candidate is an integer (S609).

When it is determined that the result obtained by dividing the frequency of the i-th candidate by the frequency of the j-th candidate is an integer, the second loop processing is stopped (S610), and it is determined that the i-th candidate is a harmonic spectrum (S611).

When it is determined that the result obtained by dividing the frequency of the i-th candidate by the frequency of the j-th candidate is not an integer, and in Step S605, when i and j are the same, the value of j is incremented by 1, and the second loop processing continues.

In Step S606, when it is determined that j is outside the range of 0 to 9, the second loop processing ends and it is determined that the i-th candidate is a fundamental frequency spectrum (S207).

In Step S608, when it is determined that i is outside the range of 0 to 9, a list of fundamental frequencies is output (S612), and the fundamental frequency calculation processing ends.

The fundamental frequency calculation method is not limited to the method described herein, and a modification may be made.

With this, the fundamental frequency of the contact pressure detection signal is calculated, whereby it becomes possible to perform determination about whether or not the pulse component is included in the contact pressure detection signal, or the like.

The determination unit 114 may perform determination about whether or not the pulse component is included in the contact pressure detection signal when the body motion signal processing unit 113 detects a plurality of fundamental frequencies for the contact pressure detection signal. When the body motion signal processing unit 113 does not detect a plurality of fundamental frequencies for the contact pressure detection signal, it may be determined that no pulse component is included in the contact pressure detection signal.

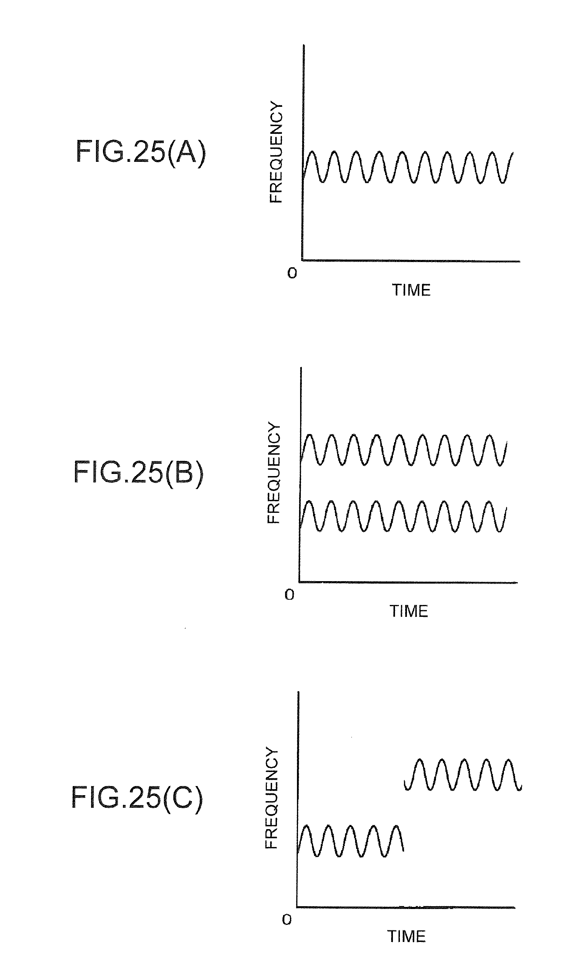

That is, when the user performs periodic movement, such as running or walking, a fundamental frequency corresponding to body motion is necessarily included in the body motion detection signal. For this reason, as in the example of Fig. 25(A), when the body motion signal processing unit 113 does not detect a plurality of fundamental frequencies for the contact pressure detection signal, that is, when a fundamental frequency other than a body motion component is not detected and only one fundamental frequency is detected, it is possible to specify that the detected fundamental frequency represents a body motion component. Accordingly, in this case, it may be determined that no pulse component is included in the contact pressure detection signal.

However, in a strict sense, the determination can be performed only when periodic movement is performed. This is because, during non-movement or the like, only the pulse component is included in the contact pressure detection signal. However, since a scene where the pulse detector 100 is actually used to acquire pulse information is often shown during movement, even if the same determination is made when periodic movement is not performed, there is no problem practically.

With this, it becomes possible to determine whether to continue the determination processing more strictly or to determine that no pulse component is included in the contact pressure detection signal according to whether or not a plurality of fundamental frequencies are included in the contact pressure detection signal.

Even when a plurality of fundamental frequencies are detected, for example, even when two fundamental frequencies are detected, it is not the case that the two fundamental frequencies necessarily represent a body motion component and a pulse component. For example, in the case of Fig. 25(B), it is considered that fundamental frequencies of body motion and pulse are detected. However, while a contact pressure is detected, the movement of the user may change from running to skipping, or the like, and in this case, two fundamental frequencies of a fundamental frequency due to running and a fundamental frequency due to skipping are detected. This is a case shown in Fig. 25(C). Since any of these fundamental frequencies represents a body motion component, the contact pressure sensor signal (contact pressure detection signal) includes no pulse component, and may be used in the body motion noise reduction processing.

Although it is difficult to determine change from running to skipping on a frequency axis in this example, if a time axis shown in Fig. 25(C) is provided, since movement will change with a certain point as a boundary, the change can be easily distinguished.

Accordingly, the body motion signal processing unit 113 may perform time frequency analysis processing for analyzing a plurality of fundamental frequencies for the contact pressure detection signal when a plurality of fundamental frequencies are detected for the contact pressure detection signal. The determination unit 114 may perform determination about whether or not the pulse component is included in the contact pressure detection signal based on the result of the time frequency analysis processing.

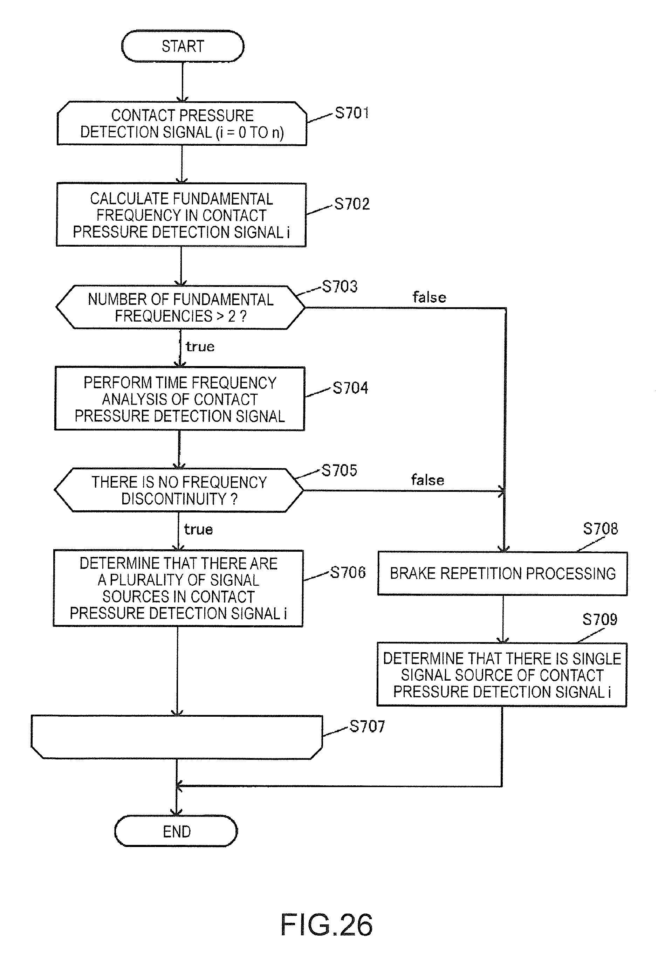

Specifically, the flow of determination processing which is performed in time frequency analysis processing will be described referring to the flowchart of Fig. 26. In the embodiment, it is assumed that the processing is performed in Step S505 of Fig. 22. However, the invention is not limited thereto. The embodiment includes a case where a plurality of contact pressure sensors 22 may be provided. For example, a contact pressure sensor based on a cuff structure and a contact pressure sensor using a piezoelectric element may be arranged separately.

First, contact pressure detection signals are acquired from (n + 1) contact pressure sensors 22, and loop processing from Step S701 to Step S707 is performed while changing i in a range of i = 0 to n (S701). Note that n is a positive integer, and for example, it is assumed that n = 2. That is, it is assumed that three contact pressure sensors 22 are provided.

Next, a fundamental frequency included in an i-th contact pressure detection signal is calculated (S702). The fundamental frequency calculation method is as shown in Fig. 24. Determination is performed about whether or not a plurality of fundamental frequencies are included in the i-th contact pressure detection signal (S703), and when it is determined that a plurality of fundamental frequencies are included in the i-th contact pressure detection signal, the contact pressure detection signal subjected to FFT for a predetermined period is divided into several sections, and time frequency analysis is performed (S704). As the time frequency analysis, for example, short-time Fourier analysis, wavelet transform, or the like is performed, and for example, change in movement, such as change in walking pitch, is detected.

Determination is performed about whether or not there is discontinuity of the fundamental frequency in a graph of Fig. 25 (C) created as the result of the time frequency analysis (S705). That is, determination is performed about whether or not there is a point at which the fundamental frequency changes rapidly, thereby performing determination about whether or not there is change in movement.

When it is determined that there is no point at which the fundamental frequency changes rapidly, it is determined that there are a plurality of signal sources in the i-th contact pressure detection signal, that is, the pulse component is included in the i-th contact pressure detection signal (S706), and the loop processing is repeated until i = n (S707). When i is outside the range of 0 to n, the processing ends.

In Step S703, when it is determined that a plurality of fundamental frequencies are not included in the i-th contact pressure detection signal, since it can be determined that only a fundamental frequency corresponding to body motion is included, the loop processing from Step S701 to Step S707 is stopped (S708), and it is determined that a signal source of the i-th contact pressure detection signal is single, that is, no pulse component is included in the i-th contact pressure detection signal (S709).

Similarly, in Step S705, when it is determined there is a point at which the fundamental frequency changes rapidly, since it can be determined that, while a plurality of fundamental frequencies are included, all fundamental frequencies are fundamental frequencies due to body motion, the loop processing is stopped (S708), and it is determined that a signal source of the i-th contact pressure detection signal is single (S709).

With this, it is possible to discriminate a contact pressure detection signal having a single signal source among the 0-th to n-th contact pressure detection signals. That is, it becomes possible to select a contact pressure detection signal including no pulse component among the contact pressure detection signals acquired from a plurality of contact pressure sensors 22, or the like. Accordingly, it becomes possible to perform appropriate body motion noise reduction processing using the contact pressure detection signal including no pulse component, or the like. Even when the contact pressure detection signal is not used, it becomes possible to perform body motion noise reduction processing using the motion detection signal, or the like.

When the body motion signal processing unit 113 detects a plurality of fundamental frequencies for the contact pressure detection signal, it is possible to accurately perform determination about whether or not the contact pressure detection signal includes the pulse component by comparing the fundamental frequencies of the contact pressure detection signal and the fundamental frequencies of the pulse wave detection signal.

Accordingly, the pulse detector 100 of the embodiment may include the pulse wave signal processing unit 111 which acquires the pulse wave detection signal from the pulse wave detection unit 10. The pulse wave signal processing unit 111 may perform the detection processing of the fundamental frequency of the pulse wave detection signal when the body motion signal processing unit 113 detects a plurality of fundamental frequencies for the contact pressure detection signal. The determination unit 114 may perform determination about whether or not the pulse component is included in the contact pressure detection signal based on the detection processing result of the fundamental frequency of the pulse wave detection signal.

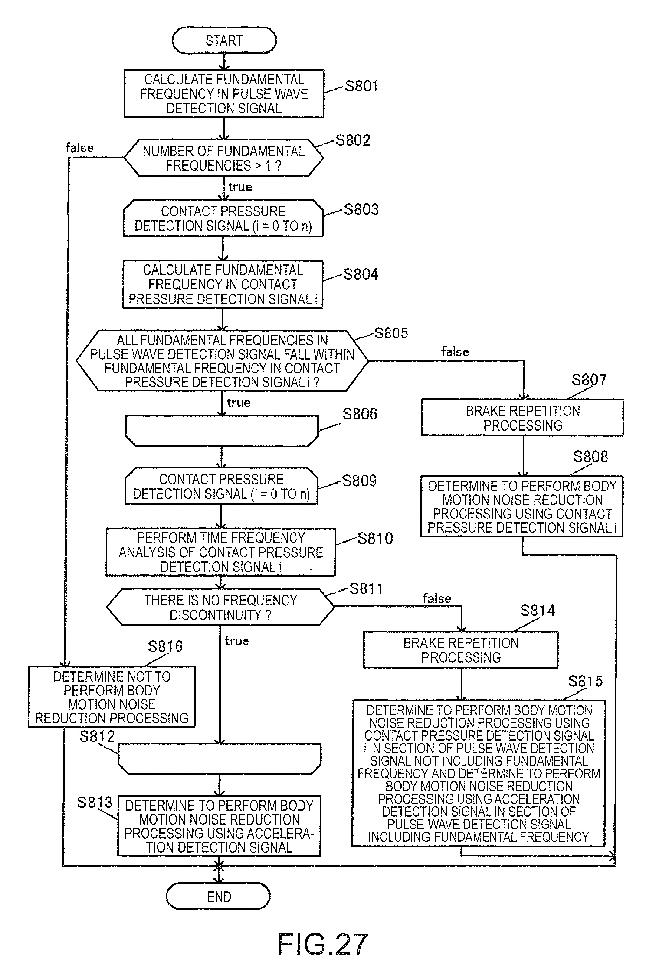

Specifically, the flow of the above-described determination processing when a pulse wave detection signal is used, instead of a body motion detection signal, will be described referring to the flowchart of Fig. 27. In the embodiment, it is assumed that the processing is performed in Step S505 of Fig. 22. However, the invention is not limited thereto.

First, the fundamental frequency included in the pulse wave detection signal is calculated (S801).

When no body motion noise component is included in the pulse wave detection signal, even if the body motion noise reduction processing is performed rather than intentionally, it is possible to obtain sufficiently accurate pulse information. For this reason, in this example, determination is performed about whether or not a plurality of fundamental frequencies are included in the pulse wave detection signal (S802), when only one fundamental frequency is included in the pulse wave detection signal, it is determined that no body motion noise component is included in the pulse wave detection signal and only a pulse component is included, it is determined not to perform body motion noise reduction processing (S816), and the processing ends.

In this case, although a modification may be made, in which the body motion noise reduction processing is performed using the motion detection signal, or the like, it is preferable not to perform body motion noise reduction processing using the contact pressure detection signal. As described above, this is because, when the user is during non-movement, only a pulse component may be included in the contact pressure detection signal, and if the body motion noise reduction processing is performed using the contact pressure detection signal, there is a concern that only the pulse component of the pulse wave detection signal is reduced.

When it is determined that a plurality of fundamental frequencies are included in the pulse wave detection signal, there is a high possibility that body motion noise is included in the pulse wave detection signal. Accordingly, since it is necessary to perform the body motion noise reduction processing, hereinafter, determination processing about which body motion detection signal is used is performed.

The contact pressure detection signals are acquired from (n + 1) contact pressure sensors 22, and the first loop processing from Step S803 to Step S806 is performed while changing i in a range of i = 0 to n (S803). Note that n is a positive integer, and for example, it is assumed that n = 2. That is, it is assumed that three contact pressure sensors 22 are provided.

Next, the fundamental frequency included in the i-th contact pressure detection signal is calculated (S804). The fundamental frequency calculation method is as shown in Fig. 24. Determination is performed about whether or not all fundamental frequencies in the pulse wave detection signal are included in the fundamental frequency of the i-th contact pressure detection signal (S805).

If there is a fundamental frequency, which is not included in the fundamental frequency of the i-th contact pressure detection signal, among the fundamental frequencies of the pulse wave detection signal, in the embodiment, it is determined that this is a pulse component. That is, it is determined that the contact pressure detection signal includes only a body motion component and no pulse component.

For this reason, when it is determined that there is a fundamental frequency, which is not included in the fundamental frequency of the i-th contact pressure detection signal, among the fundamental frequencies in the pulse wave detection signal, the first loop processing is stopped (S807), it is determined to perform the body motion noise reduction processing using the i-th contact pressure detection signal including no pulse component (S408), and the processing ends.

When it is determined that all fundamental frequencies in the pulse wave detection signal are included in the fundamental frequency of the i-th contact pressure detection signal, it is determined that the contact pressure detection signal includes a pulse component along with a body motion component, in order to search a contact pressure detection signal including no pulse component, the first loop processing is repeated (S406), when i is outside the range of 0 to n, it is determined that there is no contact pressure detection signal including no pulse component, and the processing ends.

Subsequently, after the first loop processing ends, the second loop processing from Step S809 to Step S812 is performed while changing i in a range of i = 0 to n (S809). In the second loop processing, the time frequency analysis is performed for the i-th contact pressure detection signal (S810), and determination is performed about whether or not there is no discontinuity of the fundamental frequency in a graph of Fig. 25(C) created as the result of the time frequency analysis (S811). That is, determination is performed about whether or not there is a point at which the fundamental frequency changes rapidly.

When it is determined that there is no point at which the fundamental frequency changes rapidly, it is determined that there are a plurality of signal sources in the i-th contact pressure detection signal, that is, a pulse component is constantly included in the i-th contact pressure detection signal along with a body motion component, and in order to search a contact pressure detection signal having a section with no pulse component, the second loop processing is repeated until i = n (S812). When i is outside the range of 0 to n, the second loop processing ends. Finally, the body motion noise reduction processing using the contact pressure detection signal is abandoned, it is determined to perform the body motion noise reduction processing using the acceleration detection signal (S813), and the entire processing ends.

In Step S811, when it is determined that there is a point at which the fundamental frequency changes rapidly, while a plurality of fundamental frequencies are included, there is a possibility that all of the fundamental frequencies are fundamental frequencies due to body motion depending on sections, the loop processing is stopped (S814). It is determined to perform the body motion noise reduction processing using the i-th contact pressure detection signal in a section including no fundamental frequency of the pulse wave detection signal, it is determined to perform the body motion noise reduction processing using the acceleration detection signal in a section including the fundamental frequency of the pulse wave detection signal, and the processing ends (S815).

As a specific determination method about whether or not a pulse component is included between the respective time sections of the contact pressure detection signal represented on the time axis, there is the following method. First, a signal corresponding to a portion in front of each point at which the fundamental frequency of the contact pressure detection signal is switched on the time axis of Fig. 25(C) is referred to as a first contact pressure detection signal, and a signal corresponding to a portion at the back of the switching point is referred to as a second contact pressure detection signal. Determination is performed about whether or not all fundamental frequencies in the pulse wave detection signal are included in the fundamental frequency of the first contact pressure detection signal. If all fundamental frequencies are included, it is determined that the first contact pressure detection signal includes a pulse component, and if any one fundamental frequency is not included, it is determined that the first contact pressure detection signal does not include a pulse component. The same applies to the second contact pressure detection signal.

It is of course possible to execute the processing shown in Fig. 27 in modified forms, or the like. For example, the second loop processing may not be performed, and after Step S806, it may be determined to perform body motion noise reduction processing using an acceleration detection signal, or the like.

With this, when the body motion signal processing unit 113 detects a plurality of fundamental frequencies for the contact pressure detection signal, the fundamental frequency of the contact pressure detection signal and the fundamental frequency of the pulse wave detection signal are compared with each other, making it possible to more accurately perform determination about whether or not the contact pressure detection signal includes the pulse component, or the like. It also becomes possible to perform the body motion noise reduction processing using a contact pressure detection signal with no pulse component among a plurality of contact pressure detection signals, or the like. Furthermore, even when the contact pressure detection signal is not used, it becomes possible to perform body motion noise reduction processing using a motion detection signal, or the like.

The pulse detector 100 of the embodiment may include a pulse information calculation unit 120 which calculates pulse information based on the pulse wave detection signal after the body motion noise reduction processing. The pulse information is as described above.

With this, for example, it becomes possible to present pulse information, such as a pulse rate, which is more easily understood intuitively by the user than the pulse wave detection signal, to the user, or the like. When the above-described body motion noise reduction processing is performed, it becomes possible to improve calculation precision of the pulse rate, or the like.

The embodiment may also be applied to an electronic apparatus including the above-described pulse detector 100, the pulse wave detection unit 10, and the body motion detection unit 20.

With this, the method of the embodiment may also be applied to the electronic apparatus including the pulse detector 100. The electronic apparatus is specifically a pulse monitor, and the configuration thereof may be as shown in Fig. 4(A) or 4(B) or may be a different configuration.

In the pulse detector 100, the electronic apparatus, the program, and the like of the embodiment, a part or a majority of the processing may be realized by a program.

With this, it becomes possible to realize the processing of the embodiment by a program. The program may be a program which is read and executed on a processing unit (for example, a DSP) of a device shown in Fig. 4(A) or the like.

A pulse wave detection device which is mounted on the user may have a pulse wave sensor 11, and a communication unit which performs communication of a pulse wave sensor signal from the pulse wave sensor 11 in a wired or wireless manner. In this case, the program of the embodiment is provided separately from the pulse wave detection device, and is read and executed on a processing unit (for example, a CPU) or the like of an information processing system, which receives the pulse wave sensor signal from the above-described communication unit. The information processing system may be a PC or the like which is not assumed to be mounted on the user or may be a smartphone or the like which is assumed to be mounted on (carried with) the user. A server system or the like connected to the pulse wave detection device through a network, such as Internet, may be used as the information processing system.

When the pulse wave detection device and the information processing system on which the program is executed are provided separately, a display unit which is use to present pulse information to the user is provided at an arbitrary location. For example, the display may be performed on a display unit of the information processing system, or a display unit may be provided in the pulse wave detection device and may display pulse information output from the information processing system. A display may be performed on a display unit of a different apparatus (for example, an arbitrary client device or the like when a server system is used as the information processing system).

The above-described program is recorded in an information storage medium. Here, as the information storage medium, various recording mediums, which are readably by an information processing system or the like, including an optical disc, such as a DVD or a CD, a magneto-optical disk, a hard disk (HDD), a memory, such as a nonvolatile memory or a RAM, may be assumed.

As described above, although the embodiment has been described in detail, it can be easily understood by those skilled in the art that many modifications may be made without departing from the new matter and effects of the invention. Accordingly, all the modification examples fall within the scope of the invention. For example, in the specification and the drawings, there are some terms which are presented at least once together with other terms having a broader meaning or the same meaning. Each of the terms can be replaced with the corresponding other term at any location in the specification and the drawings. All combinations of the embodiment and the modification examples fall within the scope of the invention. The configuration and operation of the pulse detector, the electronic apparatus and the program are not limited to those described in the embodiment, and various modifications may be made.

The embodiment may be realized as the following forms or application examples.

A pulse detector according to an aspect of the invention includes a body motion noise reduction unit which performs body motion noise reduction processing for reducing body motion noise component included in a pulse wave detection signal from a pulse wave detection unit having a pulse wave sensor, and a determination unit which performs processing for determining a body motion detection signal for use in the body motion noise reduction processing, in which the determination unit determines whether or not a pulse component is included in a contact pressure detection signal from a body motion detection unit having a contact pressure sensor, and performs the determination processing about whether or not to use the contact pressure detection signal as the body motion detection signal based on the determination result, and when it is determined to use the contact pressure detection signal as the body motion detection signal, the body motion noise reduction unit performs the body motion noise reduction processing based on the pulse wave detection signal and the contact pressure detection signal.

In the aspect of the invention, determination may be performed about whether or not the pulse component is included in the contact pressure detection signal, and determination may be performed about whether or not to actually use the contact pressure detection signal as the body motion detection signal in the body motion noise reduction processing based on the determination result. For this reason, only when the use of the contact pressure detection signal in the body motion noise reduction processing is valid, it becomes possible to use the contact pressure detection signal, and even when the pulse component is included in the body motion detection signal, it is possible to perform appropriate body motion noise reduction processing.

In the aspect of the invention, the determination unit may perform the determination processing using the contact pressure detection signal as the body motion detection signal when it is determined that the pulse component is not included in the contact pressure detection signal.

With this, when the pulse component is included in the contact pressure detection signal, it becomes possible to inhibit the use of the contact pressure detection signal in the body motion noise reduction processing, or the like.

In the aspect of the invention, the body motion detection unit may have a motion sensor, and the determination unit may perform the determination processing using a motion detection signal from the body motion detection unit as the body motion detection signal when it is determined that the pulse component is included in the contact pressure detection signal.

With this, even when the pulse component is included in the contact pressure detection signal, it becomes possible to perform the body motion noise reduction processing using the motion detection signal, to obtain the pulse information, and the like.

In the aspect of the invention, the pulse detector may further include a body motion signal processing unit which performs processing for detecting a fundamental frequency of the contact pressure detection signal, in which the determination unit may perform the determination processing based on a detection processing result of the fundamental frequency of the contact pressure detection signal.

With this, the fundamental frequency of the contact pressure detection signal is calculated, making it possible to perform determination about whether or not the pulse component is included in the contact pressure detection signal.

In the aspect of the invention, when the body motion signal processing unit detects a plurality of fundamental frequencies for the contact pressure detection signal, the determination unit may perform determination about whether or not the pulse component is included in the contact pressure detection signal, and may determine that the pulse component is not included in the contact pressure detection signal when the body motion signal processing unit does not detect the plurality of fundamental frequencies for the contact pressure detection signal.

With this, it becomes possible to perform determination about whether to continue the determination processing more strictly or to determine that the pulse component is not included in the contact pressure detection signal according to whether or not a plurality of fundamental frequencies are included in the contact pressure detection signal.

In the aspect of the invention, the body motion signal processing unit may perform time frequency analysis processing for analyzing the plurality of fundamental frequencies when the plurality of fundamental frequencies are detected for the contact pressure detection signal, and the determination unit may perform determination about whether or not the pulse component is included in the contact pressure detection signal based on a result of the time frequency analysis processing.

With this, for example, it becomes possible to select a contact pressure detection signal with no pulse component among the contact pressure detection signals acquired from a plurality of contact pressure sensors.

In the aspect of the invention, the pulse detector may further include a pulse wave signal processing unit which acquires a pulse wave detection signal from the pulse wave detection unit, in which the pulse wave signal processing unit may perform processing for detecting the fundamental frequency of the pulse wave detection signal when the body motion signal processing unit detects the plurality of fundamental frequencies for the contact pressure detection signal, and the determination unit may perform determination about whether or not the pulse component is included in the contact pressure detection signal based on a detection processing result of the fundamental frequency of the pulse wave detection signal.

With this, for example, when the body motion signal processing unit detects a plurality of fundamental frequencies for the contact pressure detection signal, the fundamental frequency of the contact pressure detection signal and the fundamental frequency of the pulse wave detection signal are compared with each other, making it possible to more accurately perform determination about whether or not the contact pressure detection signal includes a pulse component, or the like.

In the aspect of the invention, the pulse detector may further include a pulse information calculation unit which calculates pulse information based on the pulse wave detection signal after the body motion noise reduction processing.

With this, for example, it becomes possible to present pulse information, such as a pulse rate, which is more easily understood intuitively by the user than the pulse wave detection signal, to the user, or the like.

An electronic apparatus according to another aspect of the invention includes the pulse detector, the pulse wave detection unit, and the body motion detection unit.

According to still another aspect of the invention, there is provided a program which causes a computer to function as the above-described respective units.

### Reference Signs List

10: pulse wave detection unit, 11: pulse wave sensor, 15: filter processing unit, 16: A/D conversion unit, 20: body motion detection unit, 21: motion sensor (acceleration sensor), 22: pressure sensor (contact pressure sensor), 26: A/D conversion unit, 70 : display unit, 80 : external I/F unit, 90: storage unit, 100: processing unit (pulse detector), 110: signal processing unit, 111: pulse wave signal processing unit, 113: body motion signal processing unit, 114: determination unit, 115: body motion noise reduction unit, 119: appropriate pressing force determination unit, 120: pulse information calculation unit, 130: display control unit, 140: time measurement unit, 200: left wrist, 300: holding mechanism, 302: guide, 400: base portion, 1111: frequency analysis unit, 1112: fundamental frequency detection unit, 1131: acceleration signal processing unit, 1132: contact pressure signal processing unit, 11311: frequency analysis unit, 11312: fundamental frequency detection unit, 11321: frequency analysis unit, 11322: fundamental frequency detection unit.

## Claims

1. A pulse monitor comprising:
a pulse wave detection unit which has a pulse wave sensor configured to output a pulse wave sensor signal;
a processing unit which calculates pulse information based on the signal from the pulse wave detection unit;
a display unit which displays a processing result in the processing unit;
a storage unit which stores the processing result in the processing unit,
a holding mechanism which holds the pulse monitor on a subject,
wherein the processing unit determines whether or not a pressing force on the subject in the pulse wave detection unit is an appropriate pressing force,
the storage unit stores holding state specification information for specifying the holding state of the holding mechanism when it is determined that the pressing force on the subject in the pulse wave detection unit is the appropriate pressing force, and
the processing unit performs control for displaying the holding state specification information on the display unit.

2. The pulse monitor according to claim 1,
wherein the processing unit determines whether or not the pressing force is the appropriate pressing force based on the pulse wave sensor signal.

3. The pulse monitor according to claim 2,
wherein the processing unit determines whether or not the pressing force is the appropriate pressing force based on at least one of an AC component signal corresponding to an AC component of the pulse wave sensor signal and a DC component signal corresponding to a DC component of the pulse wave sensor signal.

4. The pulse monitor according to claim 3,
wherein the processing unit determines whether or not the pressing force is the appropriate pressing force based on the change characteristic of the AC component signal when the pressing force is changed.

5. The pulse monitor according to claim 4,
wherein the processing unit determines whether or not the pressing force is the appropriate pressing force based on the change characteristic in amplitude of the AC component signal when the pressing force is changed.

6. The pulse monitor according to claim 3,
wherein the processing unit determines whether or not the pressing force is the appropriate pressing force based on the change characteristic of the DC component signal when the pressing force is changed.

7. The pulse monitor according to claim 6,
wherein the processing unit detects an inflection point of the DC component signal based on the change characteristic of the DC component signal when the pressing force is changed and determines whether or not the pressing force is the appropriate pressing force based on the detected inflection point.

8. The pulse monitor according to any one of claims 1 to 7,
wherein the processing unit performs control for displaying, on the display unit, an instruction screen for giving an instruction to set an environment for determination about whether or not the pressing force is the appropriate pressing force.

9. The pulse monitor according to claim 8,
wherein the processing unit performs control for displaying, on the display unit, a screen for giving an instruction to stabilize body motion of the subject as the instruction screen.

10. The pulse monitor according to claim 8 or 9,
wherein the processing unit performs control for displaying, on the display unit, a screen for giving an instruction to the subject to take a given posture for determination as the instruction screen.

11. The pulse monitor according to any one of claims 8 to 10,
wherein the processing unit performs control for displaying, on the display unit, a screen for giving an instruction to the subject to stay on standby for a given time as the instruction screen.

12. The pulse monitor according to any one of claims 9 to 11,
wherein the processing unit determines whether or not the condition of the environment for determination displayed on the instruction screen is satisfied, and determines whether or not the pressing force is the appropriate pressing force when it is determined that the condition is satisfied.

13. The pulse monitor according to claim 12,
wherein the processing unit determines whether or not the condition of the environment for determination displayed on the instruction screen is satisfied based on at least one of a body motion detection signal from a body motion sensor and time measurement information from a time measurement unit, and determines whether or not the pressing force is the appropriate pressing force when it is determined that the condition is satisfied.

14. The pulse monitor according to any one of claims 1 to 13,
wherein the processing unit has a holding state specification information acquisition mode and a pulse information calculation mode, in which the pulse information is calculated, as a processing mode,
when the holding state specification information acquisition mode is set, the processing unit acquires the holding state specification information based on the determination result about whether or not the pressing force is the appropriate pressing force and stores the acquired holding state specification information in the storage unit, and
when the pulse information calculation mode is set after the holding state specification information is acquired, the processing unit performs control for reading the holding state specification information stored in the storage unit and displaying the read holding state specification information on the display unit.

15. The pulse monitor according to any one of claims 1 to 14,
wherein the holding mechanism takes first to N-th (where N is an integer equal to or greater than 2) states, in which the pressing force on the subject in the pulse wave detection unit is different, as the holding state, and
the processing unit acquires information corresponding to at least one of the first to N-th states as the holding state specification information based on a determination result about whether or not the pressing force is the appropriate pressing force in each of the first to N-th states.

16. The pulse monitor according to claim 15,
wherein, after determination about whether or not the pressing force is the appropriate pressing force in an i-th (where i is an integer which satisfies 1 ≤ i ≤ N) state, the processing unit performs control for displaying, on the display unit, an instruction screen for giving an instruction to change the holding state of the holding mechanism to a j-th (where j is an integer which satisfies 1 ≤ j ≤ N and j ≠ i) state in which the pressing force is less than the i-th state.

17. A pulse monitor comprising:
a pulse wave detection unit which has a pulse wave sensor;
a processing unit which determines whether or not a pressing force on a subject in the pulse wave detection unit is an appropriate pressing force and calculates pulse information of the subject based on a signal from the pulse wave detection unit;
a display unit which displays a processing result in the processing unit; and
a storage unit which stores the processing result in the processing unit,
wherein the processing unit performs control for displaying, on the display unit, an instruction screen for giving an instruction to set an environment for determination about whether or not the pressing force is the appropriate pressing force.

18. The pulse monitor according to claim 17,
wherein the processing unit determines whether or not the condition of the environment for determination displayed on the instruction screen is satisfied and determines whether or not the pressing force is the appropriate pressing force based on the signal from the pulse wave detection unit when it is determined that the condition is satisfied.

19. A program which causes a computer to function as:
a pulse wave detection unit which has a pulse wave sensor configured to output a pulse wave sensor signal;
a processing unit which calculates pulse information based on the signal from the pulse wave detection unit;
a display unit which displays a processing result in the processing unit; and
a storage unit which stores the processing result in the processing unit,
wherein the processing unit determines whether or not a pressing force on a subject in the pulse wave detection unit is an appropriate pressing force,
the storage unit stores holding state specification information for specifying the holding state of the holding mechanism configured to hold a pulse monitor on the subject when it is determined that the pressing force on the subject in the pulse wave detection unit is the appropriate pressing force, and
the processing unit performs control for displaying the holding state specification information on the display unit.

20. A program which causes a computer to function as:
a pulse wave detection unit having a pulse wave sensor;
a processing unit which determines whether or not a pressing force on a subject in the pulse wave detection unit is an appropriate pressing force and calculates pulse information of the subject based on a signal from the pulse wave detection unit;
a display unit which displays a processing result in the processing unit; and
a storage unit which stores the processing result in the processing unit,
wherein the processing unit performs control for displaying, on the display unit, an instruction screen for giving an instruction to set an environment for determination about whether or not the pressing force is the appropriate pressing force.

## Amended claims

### Amended claims under Art. 19.1 PCT

**1.** (Amended) A pulse monitor comprising:
a pulse wave detection unit which has a pulse wave sensor configured to output a pulse wave sensor signal;
a processing unit which calculates pulse information based on the signal from the pulse wave detection unit;
a storage unit which stores a processing result in the processing unit,
a holding mechanism which holds the pulse monitor on a subject,
wherein the processing unit determines whether or not a pressing force on the subject in the pulse wave detection unit is an appropriate pressing force, and
the storage unit stores holding state specification information for specifying the holding state of the holding mechanism when it is determined that the pressing force on the subject in the pulse wave detection unit is the appropriate pressing force.

**2.** The pulse monitor according to claim 1,
wherein the processing unit determines whether or not the pressing force is the appropriate pressing force based on the pulse wave sensor signal.

**3.** The pulse monitor according to claim 2,
wherein the processing unit determines whether or not the pressing force is the appropriate pressing force based on at least one of an AC component signal corresponding to an AC component of the pulse wave sensor signal and a DC component signal corresponding to a DC component of the pulse wave sensor signal.

**4.** The pulse monitor according to claim 3,
wherein the processing unit determines whether or not the pressing force is the appropriate pressing force based on the change characteristic of the AC component signal when the pressing force is changed.

**5.** The pulse monitor according to claim 4,
wherein the processing unit determines whether or not the pressing force is the appropriate pressing force based on the change characteristic in amplitude of the AC component signal when the pressing force is changed.

**6.** The pulse monitor according to claim 3,
wherein the processing unit determines whether or not the pressing force is the appropriate pressing force based on the change characteristic of the DC component signal when the pressing force is changed.

**7.** The pulse monitor according to claim 6,
wherein the processing unit detects an inflection point of the DC component signal based on the change characteristic of the DC component signal when the pressing force is changed and determines whether or not the pressing force is the appropriate pressing force based on the detected inflection point.

**8.** (Amended) The pulse monitor according to any one of claims 1 to 7, further comprising:
a display unit which displays the processing result in the processing unit,
wherein the processing unit performs control for displaying, on the display unit, an instruction screen for giving an instruction to set an environment for determination about whether or not the pressing force is the appropriate pressing force.

**9.** The pulse monitor according to claim 8,
wherein the processing unit performs control for displaying, on the display unit, a screen for giving an instruction to stabilize body motion of the subject as the instruction screen.

**10.** The pulse monitor according to claim 8 or 9,
wherein the processing unit performs control for displaying, on the display unit, a screen for giving an instruction to the subject to take a given posture for determination as the instruction screen.

**11.** The pulse monitor according to any one of claims 8 to 10,
wherein the processing unit performs control for displaying, on the display unit, a screen for giving an instruction to the subject to stay on standby for a given time as the instruction screen.

**12.** The pulse monitor according to any one of claims 9 to 11,
wherein the processing unit determines whether or not the condition of the environment for determination displayed on the instruction screen is satisfied, and determines whether or not the pressing force is the appropriate pressing force when it is determined that the condition is satisfied.

**13.** The pulse monitor according to claim 12,
wherein the processing unit determines whether or not the condition of the environment for determination displayed on the instruction screen is satisfied based on at least one of a body motion detection signal from a body motion sensor and time measurement information from a time measurement unit, and determines whether or not the pressing force is the appropriate pressing force when it is determined that the condition is satisfied.

**14.** The pulse monitor according to any one of claims 1 to 13,
wherein the processing unit has a holding state specification information acquisition mode and a pulse information calculation mode, in which the pulse information is calculated, as a processing mode,
when the holding state specification information acquisition mode is set, the processing unit acquires the holding state specification information based on the determination result about whether or not the pressing force is the appropriate pressing force and stores the acquired holding state specification information in the storage unit, and
when the pulse information calculation mode is set after the holding state specification information is acquired, the processing unit performs control for reading the holding state specification information stored in the storage unit and displaying the read holding state specification information on the display unit.

**15.** The pulse monitor according to any one of claims 1 to 14,
wherein the holding mechanism takes first to N-th (where N is an integer equal to or greater than 2) states, in which the pressing force on the subject in the pulse wave detection unit is different, as the holding state, and
the processing unit acquires information corresponding to at least one of the first to N-th states as the holding state specification information based on a determination result about whether or not the pressing force is the appropriate pressing force in each of the first to N-th states.

**16.** The pulse monitor according to claim 15,
wherein, after determination about whether or not the pressing force is the appropriate pressing force in an i-th (where i is an integer which satisfies 1 ≤ i ≤ N) state, the processing unit performs control for displaying, on the display unit, an instruction screen for giving an instruction to change the holding state of the holding mechanism to a j-th (where j is an integer which satisfies 1 ≤ j ≤ N and j ≠ i) state in which the pressing force is less than the i-th state.

**17.** (Amended) A program which causes a computer to function as:
a pulse wave detection unit which has a pulse wave sensor configured to output a pulse wave sensor signal;
a processing unit which calculates pulse information based on the signal from the pulse wave detection unit;
an output unit which outputs a processing result in the processing unit; and
a storage unit which stores the processing result in the processing unit,
wherein the processing unit determines whether or not a pressing force on a subject in the pulse wave detection unit is an appropriate pressing force,
the storage unit stores holding state specification information for specifying the holding state of a holding mechanism configured to hold a pulse monitor on the subject when it is determined that the pressing force on the subject in the pulse wave detection unit is the appropriate pressing force, and
the processing unit performs control for outputting the holding state specification information from the output unit.

**18.** (Amended) A pulse monitor comprising:
a pulse wave detection unit which has a pulse wave sensor configured to output a pulse wave sensor signal;
a processing unit which calculates pulse information based on the signal from the pulse wave detection unit;
an output unit which outputs a processing result in the processing unit;
a storage unit which stores the processing result in the processing unit; and
a holding mechanism which holds a pulse monitor on a subject,
wherein the processing unit determines whether or not a pressing force on the subject in the pulse wave detection unit is an appropriate pressing force,
the storage unit stores holding state specification information for specifying the holding state of the holding mechanism when it is determined that the pressing force on the subject in the pulse wave detection unit is the appropriate pressing force, and
the processing unit performs control for outputting the holding state specification information from the output unit.

**19.** (Cancelled)

**20.** (Cancelled)
